(19)

**Europäisches Patentamt**
**European Patent Office**
**Office européen des brevets**

(11) **EP 4 531 059 A2**

(12) **EUROPÄISCHE PATENTANMELDUNG**

(43) Veröffentlichungstag:
**02.04.2025 Patentblatt 2025/14**

(21) Anmeldenummer: **25157638.5**

(22) Anmeldetag: **11.11.2021**

(51) Internationale Patentklassifikation (IPC):
**H01B 1/12** (2006.01)

(52) Gemeinsame Patentklassifikation (CPC):
**A61N 1/0496; A61K 9/0009; A61K 9/06;**
**A61K 47/32; H01B 1/122**

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **27.11.2020 DE 102020131547**

(62) Dokumentnummer(n) der früheren Anmeldung(en) nach Art. 76 EPÜ:
**21819332.4 / 4 251 221**

(71) Anmelder: **Leibniz-Institut für Polymerforschung Dresden e.V.**
**01069 Dresden (DE)**

(72) Erfinder:
• **TONDERA, Christoph**
**04103 Leipzig (DE)**
• **MINEV, Ivan**
**7000 Ruse (BG)**

• **FREUDENBERG, Uwe**
**01217 Dresden (DE)**
• **WERNER, Carsten**
**01324 Dresden (DE)**
• **AKBAR, Teuku Fawzul**
**01099 Dresden (DE)**

(74) Vertreter: **Sperling, Thomas**
**Sperling, Fischer & Heyner**
**Patentanwälte**
**Tolkewitzer Straße 22**
**01277 Dresden (DE)**

Bemerkungen:
Diese Anmeldung ist am 13.02.2025 als Teilanmeldung zu der unter INID-Code 62 erwähnten Anmeldung eingereicht worden.

(54) **VERFAHREN UND MATERIAL ZUR ERFASSUNG UND BEEINFLUSSUNG EINER AUFNAHME UND ODER FREIGABE VON BIOAKTIVEN SUBSTANZEN MIT HILFE VON ELEKTRISCH LEITFÄHIGEN HYDROGELEN**

(57) Die Erfindung betrifft ein elektrisch leitfähiges Hydrogelmaterial (1), aufweisend ein aus anionisch geladenen Bausteinen (3) und ungeladenen Bausteinen ausgebildetes Polymernetzwerk (2), welches in seiner Zusammensetzung anhand von mindestens drei die anionisch geladenen Bausteine (3) definierenden Parametern, ausgewählt aus einer Gruppe von Parametern P0, P1, P2, P3, konfigurierbar ist, wobei der Parameter P0 einem Wert aus der Anzahl der ionisierten, anionischen Gruppen, unter Annahme einer 30%igen Ionisierung aller anionischen Gruppen, je Volumeneinheit des unter physiologischen Bedingungen gequollenen Hydrogelmaterials (1) entspricht, der Parameter P1 einem Wert aus der Anzahl der stark anionischen Gruppen, mit einem intrinsischen pKs-Wert kleiner als 2,5, je Volumeneinheit des unter physiologischen Bedingungen gequollenen Hydrogelmaterials (1) entspricht, der Parameter P2 einem Wert aus der Anzahl der stark anionischen Gruppen, mit einem intrinsischen pKs-Wert kleiner als 2,5, je Wiederholeinheit geteilt durch die Molmasse der Wiederholeinheit entspricht und der Parameter P3 einem Wert zur Beschreibung der Amphiphilie der anionisch geladenen Bausteine (3) entspricht, und eine elektrisch leitfähige Komponente (5), welche in dem Polymernetzwerk (2) inkorporiert ist, wobei eine elektrische Leitfähigkeit, ein elektrischer Widerstand und/oder eine elektrische Ladungsspeicherkapazität des Hydrogelmaterials (1) durch Parameterwerte einer Parameterkonfiguration des Hydrogelmaterials (1) vorgebbar ist.

Fig. 1

A: Bildung des Netzwerk 1

anionische Polymere + Vernetzermolekül = Netzwerk 1

B: Bildung des Netzwerk 2 im Netzwerk 1

Netzwerk 1 + Leitfähige monomere/Polymere = leitfähiges Hydrogel

C: Dotierung des leitfähigen Polymer im Netzwerk 2 durch die anionischen Gruppen im Netzwerk 1

C: Elektrische Ladungsmodulation im leitfähigen Polymer

EP 4 531 059 A2

**Beschreibung**

Die Erfindung betrifft ein elektrisch leitfähiges Hydrogelmaterial, mit den Merkmalen gemäß Patentanspruch 1.

Substanzfreisetzungssysteme und Substanzaufnahmesysteme auf Hydrogelbasis sind für Anwendungen in der Biotechnologie und insbesondere in der Medizin vielversprechend, da Hydrogele im Vergleich zu vielen anderen Biomaterialien dem menschlichen physiologischen Gewebe in ihrem Wassergehalt und bezüglich mechanischer Eigenschaften ähnlich sind und einen Einschluss von verschiedenen Substanzen und deren gezielte Freigabe ermöglichen. Bei Hydrogelen handelt es sich definitionsgemäß um stark hydratisierte, kovalent oder physikalisch vernetzte Polymere, welche es erlauben, Substanzen über verschiedene, nicht-kovalente Wechselwirkungen reversibel an affine Polymerbausteine des Hydrogelnetzwerks zu binden und somit entweder die Substanzen gezielt aus einem Biofluid oder einem Lebendgewebe zu entfernen, das heißt im Hydrogel zu sequestrieren, oder aus dem Hydrogel an das Biofluid oder das Lebendgewebe freizusetzen. Solche Substanzen können proteinbasierte Signalmoleküle aus der Klasse der Zytokine, Chemokine, Hormone, Neurotransmitter, Wachstumsfaktoren oder nicht-proteinbasierte niedermolekulare Wirkstoffe, sogenannte Small Molecules (engl. kleine Moleküle), welche eine oder mehrere biologische Funktionen der zuvor genannten proteinbasierten Signalmoleküle übernehmen oder auch nicht-proteinbasierte chemische/medizinische Wirkstoffe, wie antimikrobielle Wirkstoffe, Antiseptika, Farbstoffe, sein, die entweder über Ladungswechselwirkungen und/oder Hydrophobwechselwirkungen oder andere spezifische, chemische Wechselwirkungen wie Wasserstoffbrückenbindungen, reversibel an die die Affinitätvermittelnde Polymerbausteine im Hydrogel anbinden. Aus dem Stand der Technik sind Hydrogelsysteme bekannt, bei denen die physikochemischen Eigenschaften eines Hydrogels zur Sequestrierung und/oder Freisetzung für bestimmte Substanzen bereits bei der Bildung festgelegt werden müssen. Dies wird durch eine spezifische Ladungscharakteristik des Hydrogelnetzwerks wie beispielsweise aus WO 2018/162009 A2 bekannt, oder durch die gezielte Einstellung von physikalischen Eigenschaften wie Maschenweite erreicht. Es ist außerdem bekannt, kovalent gekoppelte Substanzen durch enzymatische Trigger, Licht oder variable pH-Werte kontrolliert freizusetzen, wobei zum Nachteil derartiger Hydrogelnetzwerke keine reversible Substanzbindung möglich ist. Eine kontrollierte Sequestrierung von Substanzen und somit Abreicherung aus einem Biofluid oder einer Gewebeumgebung ist somit nicht möglich.

Bei den bekannten Systemen, welche die Affinität zu den Substanzen reversibel über nicht-kovalente Wechselwirkungen realisieren, wird die Affinität und somit die Kontrolle der Sequestrierung oder Freisetzung von Substanzen durch ihre intrinsischen, das heißt bei der Bildung des Hydrogelnetzwerks aufgeprägte Netzwerkarchitektur bestimmt. Eine nachträgliche Modulation ist nur über die zuvor genannten externen Trigger Licht, pH-Wert-Änderungen oder enzymatische Spaltung möglich.

Neben intrinsisch geladenen Hydrogelen sind elektrisch leitfähige Hydrogele in der biomedizinischen Forschung beschrieben. Sie werden vorwiegend als Beschichtung für Elektrodenmaterialien, zum Beispiel für neuronale Elektroden verwendet, um deren Bioverträglichkeit und Ladungsinjektion zu optimieren. Aufgrund ihrer mechanischen Ähnlichkeit mit Geweben und Organen führt eine Verwendung von weichen hydratisierten Polymermaterialien im Vergleich zu einer Verwendung von Metallelektroden zu geringeren Abstoßungsreaktionen. Zusätzlich findet bei klassischen Metallelektroden die Ladungsinjektion, welche für die Stimulation von Geweben und Organen essentiell ist, nur an der Oberfläche der Elektroden statt. Durch Nanostrukturierung wird versucht, die Oberfläche und damit die Kontaktfläche zwischen der Elektrode und der Gewebeflüssigkeit (Biofluid) zu erhöhen, was eine erhöhte Ladungsinjektion zur Folge hat. Da das Biofluid bei elektrisch leitfähigen Hydrogelen in das gesamte Volumen des Materials eindringen kann, ist die Kontaktfläche bereits ohne weitere Strukturierung deutlich größer als bei Metallelektroden mit vergleichbarer Größe. Dies führt ebenfalls zu einer deutlich gesteigerten Ladungsinjektion. Als Maß für die Fähigkeit, Ladungen in physiologische Lösungen zu übermitteln, wird in der Literatur der Begriff Ladungsspeicherkapazität (englisch *charge storage capacity*) verwendet.

Zur Herstellung solcher leitfähigen Hydrogele werden leitfähige Substanzen wie Kohlenstoffnanoröhren und/oder elektrisch leitfähige Polymere verwendet, wobei zumeist die leitfähige Substanz in einer Hydrogel-Präkursorlösung verteilt und anschließend das Hydrogel durch Polymerisation und Vernetzung in einer weiteren von der leitfähigen Substanz unabhängigen Komponente gebildet wird. Hierbei ist als übergreifende Eigenschaft zu nennen, dass die elektrisch leitfähige Komponente zumeist hydrophob ist und daher kein Hydrogel bildet.

Aufgrund ihrer vielfältigen Einsatzmöglichkeiten und ihrer einfachen und kostengünstigen Verarbeitung werden leitfähige organische Polymere zunehmend in industriellen Anwendungen genutzt. Die Polymere arbeiten nach dem Prinzip von Halbleitern. Wichtigster Bestandteil ist ein konjugiertes $\pi$-Elektronensystem, welches sich durch das gesamte Polymerrückgrat erstreckt. Die intrinsische elektrische Leitfähigkeit dieser Polymere ist gering. Um freie Ladungsträger zu erzeugen, mit dem Ziel, die molekulare elektrische Leitfähigkeit zu steigern, werden geladene Moleküle benötigt, die Elektronen aus dem Polymersystem entfernen (p-Dotierung) oder Elektronen in das Polymersystem einbringen (n-Dotierung). Dieser Prozess wird als primäre Dotierung bezeichnet. Die meist verwendete Methode ist die p-Dotierung. Die Dotierung und daraus resultierende Interaktion von hydrophoben Polymeren mit stark geladenen hydrophilen Molekülen reduziert zusätzlich die Hydrophobizität der Polymer-Dotierungs-Komplexe und erlaubt damit die Verwendung dieser Polymere in wässrigen Lösungen oder Hydrogelen.

Neben der primären Dotierung ist auch die Anordnung der leitfähigen Polymerketten zueinander von entscheidender Bedeutung. Für den Transport von Ladungsträgern über längere Strecken müssen diese zwischen den elektrisch leitfähigen Polymerketten übertragen werden. Dafür ist eine gleichmäßige Verteilung der Polymerketten im Volumen bei gleichzeitiger räumlicher Nähe zueinander notwendig. Wässrige Lösungen von leitfähigen Polymeren und dem jeweiligen dotierenden Molekül bilden meist Suspensionen. Durch die fehlende Interaktion der leitfähigen Polymerketten untereinander resultiert eine geringe elektrische Leitfähigkeit. Durch die strukturelle Neuorganisation der leitfähigen Polymerketten zueinander (sekundäre Dotierung) kann die notwendige räumliche Nähe der Polymerketten zueinander erreicht und dadurch die Leitfähigkeit weiter gesteigert werden. Dies kann zum Beispiel durch das Erhöhen der Ionenstärke und der damit verbunden Abschirmeffekte der Ladung (charakterisiert über die Debye-Länge) erreicht werden.

Ein Beispiel mit dem leitfähigem Poly-3,4-ethylendioxythiophen (PEDOT) wird von Zhenan Bao und weiteren Autoren beschrieben (DOI: 10.1038/s41467-018-05222-4, und US20190390068A1). Wie aus dieser Schrift bekannt ist, wird aus einer Mischung von hydrophilen Polystyrensulfonat (PSS) und PEDOT (primäre Dotierung) durch physikalische Verschlaufungen des PSS und unter Verwendung von ionischen Flüssigkeiten (sekundäre Dotierung) zunächst ein schwach vernetztes Hydrogel gebildet, welches im Anschluss durch Bildung eines mit radikalischer Vernetzungsreaktion von Acrylatmonomeren gebildetes pseudo-interpenetrierendes Netzwerk mechanisch stabilisiert wird. Nach der Polymerisation resultiert ein elektrisch leitfähiges Hydrogel-Netzwerk (10.1038/s41467-018-05222-4, und US20190390068A1), wobei das sekundäre Polymer-Netzwerk lediglich der Stabilisierung und Verbesserung der mechanischen Eigenschaften des Materials dient. Die elektrischen Eigenschaften des Hydrogels resultieren allein aus dem primären, nicht kovalenten PEDOTPSS Hydrogel.

Eine weitere Methode, elektrisch leitfähige Hydrogele zu erhalten, ist aus US 9 299 476 B2 bekannt. Dem aus US 9 299 476 B2 bekannten Verfahren zufolge wird ein auf Biopolymeren basierendes Hydrogelnetzwerk, welches funktionelle anionische Gruppen trägt, auf der Oberfläche einer Elektrode gebildet und abgeschieden. Das so erhaltene primäre Netzwerk wird in einer 3,4-Ethylendioxythiophen (EDOT)-Monomere enthaltenden Lösung gequollen beziehungsweise in diese überführt und eine elektrische Polymerisation der EDOT-Monomere zu Poly-3,4-ethylendioxythiophen (PEDOT) durch den Einfluss einer elektrischen Spannung ausgelöst. Es entsteht ein pseudo-interpenetrierendes Polymernetzwerk durch die ionische Interaktion des PEDOT mit den anionischen funktionellen Gruppen des primären Netzwerkes. Diese anionischen Gruppen wirken gleichzeitig p-dotierend auf das PEDOT (primäre Dotierung) und erzeugen in Kombination ein elektrisch leitfähiges Hydrogelmaterial. Aufgrund der vorgegebenen Verteilung der kovalent im Hydrogel gebundenen anionischen Gruppen und der nachträglichen Polymerisation des PEDOT um das primäre Hydrogelnetzwerk, können aufgrund der gleichmäßigen Verteilung der PEDOT Polymerketten bereits ohne sekundäre Dotierung Hydrogele mit hoher elektrischer Leitfähigkeit erhalten werden.

Die bisher bekannten Hydrogelmaterialien ermöglichen eine Sequestrierung und/oder Freisetzung von Substanzen durch die intrinsisch aufgeprägte physikochemische Charakteristik oder zeigen im Falle von elektrisch leitfähigen Hydrogelmaterialien eine eingeschränkte Modulierbarkeit und Spezifizität für die Wechselwirkung mit bestimmten bioaktiven Substanzen.

Es ist Aufgabe der Erfindung, ein elektrisch leitfähiges Hydrogelmaterial bereitzustellen, welches hinsichtlich seiner elektrischen Eigenschaften und physikochemischen Eigenschaften modulierbar ist und eine reversible Sequestrierung und/oder Freisetzung insbesondere von positiv geladenen Gruppen tragenden Substanzen ermöglicht. Gleichzeitig soll mit diesem Material die Bindung von Substanzen durch resultierende charakteristische Änderungen der elektrischen Eigenschaften erfassbar sein.

Die Aufgabe wird durch ein elektrisch leitfähiges Hydrogelmaterial mit den Merkmalen des Patentanspruchs 1 gelöst. Weiterbildungen sind in den jeweils abhängigen Patentansprüchen angegeben. Verwendungen des elektrisch leitfähigen Hydrogelmaterials sind in den Ansprüchen 12 bis 15 angegeben.

Die Erfindung umfasst ein Verfahren zur Erfassung und Beeinflussung einer Aufnahme von bioaktiven Substanzen in einem Hydrogelmaterial und/oder Freisetzung von bioaktiven Substanzen aus dem Hydrogelmaterial, wobei das Hydrogelmaterial definitionsgemäß ein aus anionisch geladenen Bausteinen und ungeladenen Bausteinen ausgebildetes Polymernetzwerk ist, dessen Affinität für bioaktive Substanzen anhand von die anionisch geladenen Bausteine definierenden Parametern konfigurierbar ist und eine elektrisch leitfähige Komponente aufweist, deren elektrischer Widerstand und elektrische Ladungsspeicherkapazität von einer Interaktion mit Hydogel-Bausteinen und einer Bindung bioaktiver Substanzen an das Hydrogelmaterial abhängt, wobei die elektrisch leitfähige Komponente geeignet ist, die anionische Ladung des Hydrogelmaterials und dessen Affinität für bioaktive Substanzen durch den Einfluss eines elektrischen Potentials zu verändern. Bei dem Verfahren wird das vordefinierte Hydrogelmaterial mit einem Biofluid in Kontakt gebracht, wobei eine Änderung des elektrischen Widerstandes und/oder eine Änderung der Ladungsspeicherkapazität des Hydrogelmaterials erfasst und anhand der erfassten Änderung des elektrischen Widerstandes und/oder der erfassten Ladungsspeicherkapazitätsänderung eine Aufnahme von bioaktiven Substanzen in das Hydrogelmaterial oder eine Freisetzung von bioaktiven Substanzen aus dem Hydrogelmaterial in das Biofluid ermittelt wird, und/oder wobei eine Konzentration von bioaktiven Substanzen in dem Biofluid und/oder eine Konzentration von bioaktiven Substanzen in dem Hydrogelmaterial durch ein auf das Hydrogelmaterial wirkendes elektrisches Potential beeinflusst wird.

Das aus anionisch geladenen Bausteinen und ungeladenen Bausteinen gebildete Polymernetzwerk ist ein anionisch geladenes Polymernetzwerk. Das anionisch geladene Polymernetzwerk ist anhand von Parametern, welche die anionisch geladenen Bausteine definieren, konfigurierbar.

Das definitionsgemäße Hydrogelmaterial, welches elektrisch leitfähig ist, wird der Einfachheit halber nachfolgend als Hydrogelmaterial bezeichnet.

Als Biofluid sind im Sinne der Erfindung physiologische Lösungen, Zellkulturen und Lebendgewebe zu verstehen. Die Handlungsanweisung Kontaktieren des Hydrogelmaterials mit einem Biofluid kann daher als eine Kontaktierung durch Eintauchen in eine physiologische Lösung oder als eine Flächenkontaktierung des Hydrogelmaterials mit einem Lebendgewebe *in vivo* und *in vitro* verstanden werden.

Als bioaktive Substanzen werden im Sinne der Erfindung proteinbasierte und nicht proteinbasierte bioaktive Substanzen, Wirkstoffe sowie Small Molecules, welche Signaleigenschaften von Zytokinen, Chemokinen, Hormonen, Neurotransmittern oder Wachstumsfaktoren aufweisen sowie weitere biologische Wirkungen verursachen, verstanden. Bioaktive Substanzen können insbesondere pharmazeutische Wirkstoffe sein. Für alle zuvor genannten bioaktiven Substanzen gilt als übergreifendes Merkmal ein Molekulargewicht von kleiner gleich 70 kDa.

Das erfindungsgemäße Verfahren umfasst eine Erfassung einer Aufnahme von bioaktiven Substanzen in einem Hydrogelmaterial und/oder Freisetzung von bioaktiven Substanzen aus dem Hydrogelmaterial sowie eine Beeinflussung einer Aufnahme von bioaktiven Substanzen in einem Hydrogelmaterial und/oder Freisetzung von bioaktiven Substanzen aus dem Hydrogelmaterial. Es kann somit eine Erfassung und Beeinflussung von bioaktiven Substanzen oder - gemäß der Alternative - eine Erfassung oder Beeinflussung von bioaktiven Substanzen durchgeführt werden.

Dass zur Erfassung einer Aufnahme von bioaktiven Substanzen in dem Hydrogelmaterial und/oder einer Freisetzung von bioaktiven Substanzen aus dem Hydrogelmaterial eine Änderung des elektrischen Widerstands des Hydrogelmaterials und/oder eine elektrische Ladungskapazitätsänderung des Hydrogelmaterials erfasst wird/werden, liegt der Erkenntnis zugrunde, dass die Änderung des elektrischen Widerstands des Hydrogelmaterials und/oder eine elektrische Ladungskapazitätsänderung des Hydrogelmaterials durch die Bindung bioaktiver Substanzen an das Hydrogelmaterial beeinflusst wird. So wird der elektrische Widerstand des Hydrogelmaterials infolge der Sequestrierung und Bindung an oder in dem Hydrogelmaterial erhöht, wobei die elektrische Leitfähigkeit des Hydrogelmaterials durch die Beladung der anionischen Gruppen mit bioaktiven Substanzen sinkt. Im Umkehrschluss wird der elektrische Widerstand des Hydrogelmaterials verringert, wenn bioaktive Substanzen aus oder von dem Hydrogelmaterial gelöst werden, wobei die elektrische Leitfähigkeit des Hydrogelmaterials ansteigt.

Die Berechnung der Ladungsspeicherkapazität erfolgt anhand der Messung der zyklischen Voltammetrie. Dafür wird in einem Drei-Elektroden-Aufbau der Stromfluss zwischen Arbeitselektrode (Hydrogelmaterial) und Gegenelektrode (Kohlenstoff-Elektrode) gemessen, während in fünf zyklischen Durchläufen das angelegte elektrische Potential von -0,6 bis 0,8 V (Potential zwischen Arbeitselektrode und Ag/AgCl-Referenzelektrode) variiert wird. Die Scan-Rate beträgt 50 mV/s. Anschließend wird der negative Teil der Fläche unter der Kurve integriert (MultiTrace 4.3, PalmSens 4) und daraus die Ladungsspeicherkapazität mit nachfolgender Formel berechnet:

$$Ladungsspeicherkapazit\ddot{a}t = \frac{Stromst\ddot{a}rke\ I * Spannung\ U}{Scan - Rate}$$

Als Integral gibt die Software den Wert I * U in der Einheit [A] * [V] aus. Dabei gilt [A] = [C/s]. Durch die Division der Scan-Rate [V/s] resultiert die Ladung [C], welche durch das Material übertragen wird. Diese wird anschließend in das Verhältnis zum Volumen gesetzt, da die Grenzfläche zum umgebenden Medium das komplette Volumen des Hydrogels betrifft und dadurch eine Berechnung der Oberfläche/Kontaktfläche nicht möglich ist.

Zur Erfassung einer Aufnahme von bioaktiven Substanzen in dem Hydrogelmaterial und/oder einer Freisetzung von bioaktiven Substanzen aus dem Hydrogelmaterial kann alternativ eine Änderung der elektrischen Leitfähigkeit des Hydrogelmaterials bestimmt werden, wobei anhand der Änderung der elektrischen Leitfähigkeit eine Aufnahme von bioaktiven Substanzen in dem Hydrogelmaterial und/oder eine Freisetzung von bioaktiven Substanzen aus dem Hydrogelmaterial ermittelt wird. Die Bindung bioaktiver Substanzen im elektrisch leitfähigen Hydrogelmaterial erfolgt über nichtkovalente Wechselwirkungen der bioaktiven Substanzen mit den Polymerketten des Hydrogelmaterials. Dabei spielen insbesondere ionische Wechselwirkungen eine wichtige Rolle. Durch die Interaktion von anionischen und kationischen Gruppen der gebundenen bioaktiven Substanzen mit dem anionische Gruppen tragenden Polymernetzwerk des Hydrogelmaterials sowie dem kationischen, elektrisch leitfähigen Polymer, erfolgt eine Änderung der Dotierung des leitfähigen Polymers. Die anionischen Gruppen in den gebundenen Substanzen können dabei zur Dotierung beitragen und die Leitfähigkeit erhöhen. Kationische Gruppen hingegen kompensieren negative Ladungen der anionischen Polymerkomponente und haben dadurch einen negativen Einfluss auf die Dotierung. Zusätzlich können hydrophobe Bereiche der biokativen Substanzen die Interaktion der PEDOT Polymerketten zueinander beeinflussen. In Abhängigkeit von dem Molekültyp der gebundenen bioaktiven Substanzen sowie der Konzentration der bioaktiven Substanzen findet somit eine spezifische Änderung der elektrischen Eigenschaften des Hydrogelmaterials statt.

Eine Aufnahme und/oder Freisetzung von bioaktiven Substanzen kann als Konzentrationsänderung von bioaktiven Substanzen in dem Hydrogelmaterial verstanden werden. Ferner kann vorgesehen sein, dass einem diskreten Leitfähigkeitswert, einem diskreten elektrischen Widerstandswert oder einem diskreten Wert der elektrischen Ladungsspeicherkapazität eine diskrete Konzentration einer bioaktiven Substanz zugeordnet wird.

Der elektrische Widerstand des Hydrogelmaterials kann als Gleichstromwiderstand oder als Impedanz ermittelt werden. Für die Impedanzerfassung kann ein Frequenzbereich von 0,01 Hz bis 1 MHz vorgegeben sein. Es kann vorgesehen werden, dass zur Erfassung einer Bindung von bioaktiven Substanzen an oder in dem Hydrogel eine Veränderung der Impedanz des Hydrogelmaterials bei zumindest einer Frequenz im Bereich von 0,1 Hz bis 1 MHz gemessen wird. Dabei kann weiterhin eine Veränderung der Ladungsspeicherkapazität berücksichtigt werden.

Zur Beeinflussung einer Konzentration von bioaktiven Substanzen in dem Biofluid und/oder einer Konzentration von bioaktiven Substanzen in dem Hydrogelmaterial wird das Hydrogelmaterial mit einem elektrischen Potential beaufschlagt. Durch den Einfluss eines elektrischen Potentials auf das Hydrogelmaterial wird die anionische Ladung des Hydrogelmaterials und dessen Affinität für bioaktive Substanzen geändert, so dass infolge einer Potentialänderung eine Bindung von bioaktiven Substanzen in oder an dem Hydrogelmaterial beeinflusst wird.

Eine Freisetzung von bioaktiven Substanzen aus dem Hydrogelmaterial setzt voraus, dass eine bioaktive Substanz vor der Kontaktierung mit dem Biofluid an oder in dem Hydrogelmaterial gebunden ist. Es kann daher vorgesehen werden, dass zur Freisetzung von bioaktiven Substanzen das Hydrogelmaterial vor der Kontaktierung mit dem Biofluid elektrisch oder chemisch mit einer vorgegebenen Konzentration der vorgegebenen bioaktiven Substanz beladen wird.

Gemäß einer Ausführungsvariante des erfindungsgemäßen Verfahrens ist vorgesehen, dass das Polymernetzwerk in seiner Zusammensetzung anhand von mindestens drei die anionisch geladenen Bausteine definierenden Parametern, ausgewählt aus einer Gruppe von Parametern P0, P1, P2, P3, konfigurierbar ist, wobei der Parameter P0 einem Wert aus der Anzahl der ionisierten, anionischen Gruppen, unter Annahme einer 30%igen Ionisierung aller anionischen Gruppen, je Volumeneinheit des unter physiologischen Bedingungen gequollenen Hydrogelmaterials entspricht, der Parameter P1 einem Wert aus der Anzahl der stark anionischen Gruppen, mit einem intrinsischen pKs-Wert kleiner als 2,5, je Volumeneinheit des unter physiologischen Bedingungen gequollenen Hydrogelmaterials entspricht, der Parameter P2 einem Wert aus der Anzahl der stark anionischen Gruppen, mit einem intrinsischen pKs-Wert kleiner als 2,5, je Wiederholeinheit geteilt durch die Molmasse der Wiederholeinheit entspricht und der Parameter P3 einem Wert zur Beschreibung der Amphiphilie der anionischen Bausteine entspricht, wobei ein elektrischer Widerstand und/oder eine elektrische Ladungsspeicherkapazität des Hydrogelmaterials durch Parameterwerte einer Parameterkonfiguration des Hydrogelmaterials vorgegeben wird. Eine detaillierte Definition der Parameterwerte und deren Bestimmung ist der Beschreibung weiter unten zu entnehmen.

Eine Wechselwirkung von bioaktiven Substanzen kann weiterhin auf dem substanzspezifischen Wert Pp beruhen, welcher aus dem Verhältnis der Nettoladung einer bioaktiven Substanz und der für Wasser zugänglichen Oberfläche der bioaktiven Substanz berechnet wird. Für proteinbasierte Substanzen ist eine beliebige Proteinstruktur in der Protein Data Bank (PDB, http://www.rcsb.org/) verfügbar. Die Nettoladung der ausgewählten Proteinstruktur wird mit dem Delphi Web Server (http://compbio.clemson.edu/sapp/delphi_webserver/) mit Standardeinstellungen bei pH 7 berechnet. Die dem Lösungsmittel Wasser zugängliche Proteinoberfläche wird mit der PyMol-Software (www.pymol.org) unter Nutzung von einem Lösungsmittelradius von Wasser von 1.4 Å berechnet. Dann erfolgt die Berechnung von Pp aus der Nettoladung dividiert durch die dem Lösungsmittel Wasser zugängliche Proteinoberfläche multipliziert mit einem Faktor von 1000000, um die Einheit $10^{-6}$ x [$1/A^2$ bzw. $A^{-2}$] zu erhalten. Für nicht-proteinbasierte Substanzen wird die aus der chemischen Struktur ableitbare Nettoladung, welche dem Überschuss anionischer oder kationischer Gruppen entspricht, und die durch das Lösungsmittel Wasser zugängliche Moleküloberfläche, welche in Analogie zur Bildungsvorschrift für den Parameter P3 durch Nutzung der ChemDraw19.0 und ChemAxon MarvinSketch 19.21 Software abgeleitet wurde, berechnet. Der erhaltene Wert wird mit einem Faktor von 1000000 multipliziert, um die Einheit $10^{-6}$ x [$1/A^2$ bzw. $A^{-2}$] zu erhalten.

Eine Beladung des Hydrogelmaterials, also die Immobilisierung einer vorgegebenen Konzentration einer bioaktiven Substanz in oder an dem Hydrogelmaterial, kann auf unterschiedliche Weise erfolgen. Gemäß einer ersten Methode, die auch als erste Beladungsmethode bezeichnet werden kann, wird eine vorgegebene Konzentration der zur Bindung vorgesehenen bioaktiven Substanz oder Substanzen mit den anionisch geladenen Hydrogelbausteinen gemischt und dabei in das Polymernetzwerk integriert. Bei der nachfolgenden Bildung der elektrisch leitfähigen Hydrogelmaterialien durch Inkorporation der elektrisch leitfähigen Komponente sind die bioaktiven Substanzen dann bereits gemäß der vorgegebenen Konzentration in dem Polymernetzwerk enthalten. Bei diesem Vorgehen ist von Vorteil, dass die gesamte Menge der bioaktiven Substanz beziehungsweise der bioaktiven Substanzen nach der Polymernetzwerkbildung quantitativ im Hydrogelmaterial enthalten ist. Mit anderen Worten, die Beladung der bioaktiven Substanz oder der bioaktiven Substanzen erfolgt unabhängig von den Parametern P0, P1, P2, P3 und unabhängig von dem substanzspezifischen Wert Pp. Die bei der Hydrogelmaterialbildung herrschenden Reaktionsbedingungen können einen ungünstigen Einfluss auf die Struktur der bioaktiven Substanzen haben, weshalb nicht alle bioaktiven Substanzen für eine Immobilisierung nach der ersten Beladungsmethode geeignet sind.

Gemäß einer zweiten Methode, die auch als zweite Beladungsmethode bezeichnet werden kann, wird das elektrisch leitfähige Hydrogelmaterial mit einer vorgegebenen Parameterkonfiguration und vorgegebenen Parameterwerten gebildet und das elektrisch leitfähige Hydrogelmaterial im Anschluss mit einer wässrigen Lösung oder einem Biofluid als Beladungslösung mit einer vorgegebenen Substanzkonzentration für eine vorgegebene Zeitdauer in Kontakt gebracht. Dabei werden die in Lösung befindlichen bioaktiven Substanzen aus der wässrigen Lösung oder dem Biofluid in Abhängigkeit der Parameter P0, P1, P2, P3 in das Hydrogelmaterial aufgenommen und gebunden. Abschließend wird das beladene Hydrogelmaterial aus der Beladungslösung entfernt. Die an oder in dem Hydrogelmaterial immobilisierten bioaktiven Substanzen können dann durch den Einfluss eines elektrischen Potentials an eine Umgebung, vorzugsweise ein Biofluid oder ein Lebendgewebe, freigesetzt werden. Dabei besteht durch den Einfluss des elektrischen Potentials weiter die Möglichkeit, dass bioaktive Substanzen aus der Umgebung des Hydrogelmaterials in das Hydrogelmaterial sequestriert werden.

Gemäß einer dritten Methode zur Beladung, welche auch als dritte Beladungsmethode bezeichnet werden kann, wird ein vorgegebenes elektrisch leitfähiges Hydrogelmaterial, welches eine vorgegebene Parameterkonfiguration mit vorgegebenen Parameterwerten aufweist, mit einer wässrigen Lösung oder einem Biofluid als Beladungslösung mit einer vorgegebenen Substanzkonzentration für eine vorgegebene Zeitdauer in Kontakt gebracht und dabei dem Einfluss eines elektrischen Potentials ausgesetzt. Dabei werden bioaktive Substanzen in Abhängigkeit der vorgegebenen Parameterwerte aus der Beladungslösung in das Hydrogelmaterial aufgenommen (sequestriert). Es kann erforderlich sein, dass das auf das Hydrogelmaterial wirkende Potential aufrecht erhalten werden muss, um die Bindung der bioaktiven Substanzen konstant zu halten. Andernfalls, wenn das Potential beziehungsweise der Strom verändert wird, können die bioaktiven Substanzen aus dem Hydrogelmaterial freigesetzt werden. Wesentlich ist dabei, dass die Sequestrierung oder Freigabe von bioaktiven Substanzen neben der Beeinflussung durch ein elektrisches Potential auch auf einer durch die Paramter P0, P1, P2, P3, Pp vorgegebenen Affinität des Hydrogelmaterials für bestimmte bioaktive Substanzen beruht.

Es hat sich gezeigt, dass eine die elektrische Leitfähigkeit des Hydrogelmaterials beeinflussende Strukturbildung und Verteilung der elektrisch leitfähigen Komponente in dem Hydrogelmaterial durch die Parameterwerte einer Parameterkonfiguration beeinflusst werden kann. Es kann daher vorgesehen werden, dass ein Hydrogelmaterial vorgegeben wird, welches eine vorgegebene elektrische Leitfähigkeit aufgrund einer vorgegeben Parameterkonfiguration mit vorgegeben Parameterwerten aufweist.

Bei dem Verfahren kann weiter vorgesehen sein, dass das Hydrogelmaterial zur Aufnahme von bioaktiven Substanzen mit einem elektrischen Potential, gegenüber einer Ag/AgCl Referenzelektrode, im Bereich von 1 mV bis 1000 mV, vorzugsweise im Bereich von 400 mV bis 600 mV beaufschlagt wird. Zur Freigabe von bioaktiven Substanzen kann das Hydrogelmaterial mit einem elektrischen Potential, gegenüber einer Ag/AgCl Referenzelektrode, im Bereich von -1 mV bis -1000 mV, vorzugsweise in einem Bereich von -400 mV bis -600 mV beaufschlagt werden.

Das Hydrogelmaterial kann zur Aufnahme von bioaktiven Substanzen mit einem konstanten elektrischen Strom größer als 0 mA beaufschlagt werden, wobei die Richtung des elektrischen Stromflusses zur Freigabe von bioaktiven Substanzen geändert wird.

Die Erfindung umfasst weiter ein elektrisch leitfähiges Hydrogelmaterial, welches zur Durchführung des vorstehend beschriebenen Verfahrens geeignet ist. Das erfindungsgemäße Hydrogelmaterial weist ein aus anionisch geladenen Bausteinen und ungeladenen Bausteinen ausgebildetes Polymernetzwerk auf oder besteht aus einem solchen Polymernetzwerk, welches in seiner Zusammensetzung anhand von mindestens drei die anionisch geladenen Bausteine definierenden Parametern, ausgewählt aus einer Gruppe von Parametern P0, P1, P2, P3, konfigurierbar ist, wobei der Parameter P0 einem Wert aus der Anzahl der ionisierten, anionischen Gruppen, unter Annahme einer 30%igen Ionisierung aller anionischen Gruppen, je Volumeneinheit des unter physiologischen Bedingungen gequollenen Hydrogelmaterials entspricht, der Parameter P1 einem Wert aus der Anzahl der stark anionischen Gruppen, mit einem intrinsischen pKs-Wert kleiner als 2,5, je Volumeneinheit des unter physiologischen Bedingungen gequollenen Hydrogelmaterials entspricht, der Parameter P2 einem Wert aus der Anzahl der stark anionischen Gruppen, mit einem intrinsischen pKs-Wert kleiner als 2,5, je Wiederholeinheit geteilt durch die Molmasse der Wiederholeinheit entspricht und der Parameter P3 einem Wert zur Beschreibung der Amphiphilie der anionischen Bausteine entspricht. Weiterhin weist das Hydrogelmaterial eine elektrisch leitfähige Komponente auf, welche in das Polymernetzwerk inkorporiert ist, wobei eine elektrische Leitfähigkeit, ein elektrischer Widerstand und/oder eine elektrische Ladungsspeicherkapazität des Hydrogelmaterials durch Parameterwerte einer Parameterkonfiguration des Hydrogelmaterials vorgebbar ist.

Die Parameter P0 bis P3 sind anhand der nachfolgenden Festlegungen und Bildungsvorschriften näher definiert:

Parameter P0: der Wert für P0, welcher in $\mu$mol/ml angegeben werden kann, entspricht 30 % der Gesamtanzahl anionischer Gruppen bezogen auf das unter physiologischen Bedingungen (0,154 mmol/l NaCl, pH gepuffert auf 7,4) gequollene Hydrogelvolumen. Die Berechnung kann beispielsweise aus der Polymerkonzentration der Hydrogelbausteine unter Quellung in physiologischer Lösung berechnet werden.

Parameter P1: der Wert für P1, welcher in $\mu$mol/ml angegeben werden kann, entspricht der Anzahl der stark anionischen Gruppen, welche einen pKs-Wert kleiner als 2,5, aufweisen, bezogen auf das unter physiologischen Bedingungen (0,154 mmol/l NaCl, pH gepuffert auf 7,4) gequolle Hydrogelvolumen.

Parameter P2: der Wert für P2, welcher in mmol/(g/mol) angegeben werden kann, entspricht der Anzahl der stark anionischen Gruppen mit einem pKs-Wert kleiner als 2,5 je anionisch geladenen Bausteins geteilt durch das jeweilige Molekulargewicht des anionischen Bausteins.

Parameter P3: Der Parameter P3, welcher die Amphiphilie der anionischen Bausteine beschreibt, wird durch Division des Verteilungskoeffizienten Oktanol/Wasser (LogP-Wert) des anionischen Bausteins durch die dem Lösungsmittel Wasser zugängliche Oberfläche des anionischen Bausteins berechnet. Dies kann unter Zuhilfenahme der Software ChemDraw19.0 und ChemAxon MarvinSketch 19.21 wie folgt erfolgen: Unter Nutzung der Software ChemDraw19.0 wird jeder anionische Baustein mit einer Länge des Polymerrückgrates von 22 Kohlenstoffatomen beziehungsweise bei zuckerbasierten Strukturen mit insgesamt 2 Disaccharideinheiten als komplette chemische Struktur dargestellt. Anschließend wird durch Nutzung der Softwareanwendung ChemAxon MarvinSketch 19.21 der Verteilungskoeffizient Oktanol/Wasser (LogP-Wert) über Einlesen der durch ChemDraw19.0 dargestellten Strukturformeln berechnet und die durch das Lösungsmittel Wasser zugängliche Oberfläche mit einem Lösungsmittelradius von 1.4 Å berechnet. Der erhaltene Wert wird mit einem Faktor von 1000 multipliziert, um die Einheit $10^{-3} \times [1/A^2$ bzw. $A^{-2}]$ zu erhalten.

Das erfindungsgemäße Hydrogelmaterial ist wesentlicher Bestandteil zur Durchführung des erfindungsgemäßen Verfahrens. Die das erfindungsgemäße Hydrogel betreffenden Merkmale können daher zur näheren Erläuterung des erfindungsgemäßen Verfahrens, insbesondere zur Definition des Hydrogelmaterials, verwendet werden und umgekehrt. Das erfindungsgemäße Hydrogelmaterial, basiert auf einem anionische Gruppen tragenden Polymernetzwerk, welches durch die Parameter P0, P1, P2, P3 in seinen Eigenschaften definiert ist. Anhand dieser Parameter P0 bis P3 wird die Bildung eines (pseudo)-interpenetrierenden Netzwerks aus elektrisch leitfähigen Komponenten und somit die elektrischen Eigenschaften des sich bildenden elektrisch leitfähigen Hydrogelmaterials kontrolliert. Vorteilhafterweise kann das elektrisch leitfähige Hydrogelmaterial durch Anlegen eines elektrischen Potentials beziehungsweise durch den Einfluss eines elektrischen Potentials stufenlos in seinen Ladungseigenschaften moduliert werden, wobei die Modulation der elektrischen Ladungseigenschaften in Zusammenhang mit den Parametern P0, P1, P2, P3 eine Affinität des elektrisch leitfähigen Hydrogelmaterials zu bioaktiven Substanzen kontrolliert und sich somit die Abreicherung von Substanzen aus einem mit dem leitfähigen Hydrogel in Kontakt stehenden Biofluid (Sequestrierung) oder die Freisetzung von bioaktiven Substanzen aus dem Hydrogelmaterial in das Biofluid stufenlos, reversibel und in Echtzeit in direkter Abhängigkeit zum angelegten elektrischen Potential regulieren lassen.
Durch die Verwendung von verschiedenen, anionische Gruppen tragenden geladenen Bausteinen sowie die Variation deren Konzentration im Hydrogelmaterial und der Anzahl und Dichte an stark anionischen Gruppen entlang der Polymerkette der ungeladenen Bausteine, können nach den Parametern P0-P3 verschieden konfigurierte Hydrogelmaterialien mit gleichem Vernetzungsgrad und Feststoffgehalt synthetisiert werden. Zur Erzeugung von elektrisch leitfähigen Hydrogelmaterialien wird als elektrisch leitfähige Komponente vorzugsweise das Polymer PEDOT chemisch als pseudo-interpenetrierendes Netzwerk um die primären Hydrogelnetzwerke polymerisiert. Die erhaltenen Hydrogelmaterialien unterscheiden sich in ihren elektrischen Eigenschaften sowie in der Sequestrierung und Freisetzung von bioaktiven Substanzen in Abhängigkeit der konfigurierten Parameter beziehungsweise Parameterwerte und deren Konstellation.
Der Parameter P0, der alle tatsächlich ionisierten anionischen Gruppen unabhängig ihres intrinsischen pKa im gequollenen Hydrogel beschreibt, übt dabei im Vergleich zum Parameter P1 etwas geringeren Einfluss auf die elektrischen Eigenschaften des resultierenden Hydrogelmaterials aus. Die in dem Paramter P0 enthaltenen schwach anionischen Gruppen stellen keine effektive Dotierung für PEDOT dar, wodurch die elektrische Leitfähigkeit beziehungsweise der elektrische Widerstand im Vergleich zu dem Paramter P1, welcher die stark aziden Gruppen mit einem pKa < 2,5 beschreibt, beeinflusst wird. Die integrale Anzahl an stark negativ geladenen anionischen Gruppen (P1) dagegen beeinflusst direkt die elektrische Leitfähigkeit beziehungsweise den elektrischen Widerstand. Die minimale elektrische Leitfähigkeit wird bei einem vollständig undotierten Hydrogelmaterial (für P1 = 0) beziehungsweise ohne eine elektrisch leitfähige Komponente erreicht. Je höher die Anzahl stark geladener anionischer Gruppen im Hydrogelmaterial ist, desto höher ist die elektrische Leitfähigkeit und desto geringer ist der elektrische Widerstand. Die Ursache liegt in der Erzeugung der freien Ladungsträger durch die p-Dotierung des PEDOT. Je größer die Anzahl an dotierenden Einheiten ist, desto mehr freie Ladungsträger können gebildet werden. Für die lokale Ladungsdichte der stark anionischen Gruppen (P2) lässt sich ebenfalls eine direkte Beeinflussung der elektrischen Eigenschaften erwarten. Die Verringerung der lokalen Ladungsdichte bei gleichbleibenden P0 und P1 führt zu einer Verringerung der elektrischen Leitfähigkeit der Hydrogelmaterialien. Die Begründung liegt in der lokalen elektrostatischen Interaktion der negativ geladenen Polymere mit den positiv geladenen PEDOT Polymerketten. Eine zu geringe lokale negative Ladung führt zu einer schwächeren Interaktion der positiv geladenen PEDOT Polymerketten mit dem anionischen Polymer beziehungsweise der anionisch geladenen Bausteine. Dadurch wird eine Dotierung erschwert, was zu geringeren elektrischen Leitfähigkeiten führt. Auch bei einem moderaten Wert für P1 und zu geringem Wert für P2 lässt sich dadurch vermutlich keine gesteigerte elektrische Leitfähigkeit zu undotierten Hydrogelmaterialien beobachten. Durch den Parameter P3 lässt sich ebenfalls die Interaktion zwischen dem amphiphilen anionischen Polymer und dem hydrophoben, elektrisch leitfähigem Polymer (PEDOT) konfigurieren. Durch eine erhöhte Hydrophobizität der anionisch geladenen Bausteine lassen sich bereits bei deutlich geringerem P1-Wert gesteigerte elektrische Leitfähigkeiten erzeugen. Der Grund ist die hohe Affinität der hydrophoben PEDOT-Einheiten zu den hydrophoben Gruppen auf dem anionisch geladenen Baustein, welche die Dotierung positiv beeinflusst. Zusätzlich erleichtern die hydrophoben Gruppen bereits während der PEDOT Synthese ein besseres Eindringen der Monomereinheiten (EDOT) in das Polymernetzwerk und dadurch eine bessere Einlagerung der PEDOT Ketten.

Für eine spannungsabhängige, beziehungsweise stromabhängige Sequestrierung und Freisetzung von Substanzen spielen vorwiegend die integrale (P0 oder P1) und lokale Ladungsdichte (P2) bei den verschiedenen angelegten Spannungen/Strömen im gesamten Hydrogelmaterial eine Rolle. Ohne angelegte Spannung kompensiert die positive Ladung des PEDOT einen Teil der negativen Ladungen der anionisch geladenen Bausteine. Dadurch lassen sich im Vergleich zu Hydrogelmaterialien, welche aus einem Polymernetzwerk ohne elektrisch leitfähige Komponente bestehen, im größeren Maße auch negative geladene Moleküle binden. In Abhängigkeit der Affinität der bioaktiven Substanzen zum anionischen Polymer, das heißt zum anionisch geladenen Baustein, ist die Bindung positiv geladener bioaktiver Substanzen ebenfalls möglich. Durch die Konfiguration der Parameter P0, P1, P2, P3 lässt sich bei konstanten PEDOT-Polymerisationsbedingungen das Verhältnis der positiven Ladungen des PEDOT und der anionischen Gruppen des geladenen Bausteins einstellen. Dadurch kann eine Sequestrierung (Aufnahme) von positiv beziehungsweise negativ geladenen bioaktiven Substanzen ermöglicht werden. Durch das Anlegen einer Spannung kann zusätzlich die Ladung des PEDOT von neutral bis 66 % positiver Ladungen pro Monomereinheit eingestellt werden. Dadurch lässt sich die Bindung zusätzlich stufenlos einstellen. Die Freisetzung der gebundenen bioaktiven Substanzen lässt sich ebenfalls in Abhängigkeit der konfigurierten Parameter P0, P1, P2, P3 des Polymernetzwerks in Kombination mit der Ladung des PEDOT, also der elektrisch leitfähigen Komponente, einstellen. Zusätzlich kann auch eine stufenlose Konfiguration durch das Einstellen der Ladung des PEDOTs durch Anlegen einer elektrischen Spannung beziehungsweise eines elektrischen Stroms erfolgen. Dadurch kann eine reduzierte oder gesteigerte Freisetzung von verschieden geladenen bioaktiven Substanzen stufenlos eingestellt werden.

Die anionisch geladenen Bausteine können aus einer Gruppe, enthaltend Poly(Acrylsäure-co-4-acrylamidomethylbenzensulfonsäure), Poly(Acrylsäure-co-Acrylamidoethansulfonsäure), Poly(Acrylsäure-co-Acrylamidoethanhydrogensulfat), Poly(4-Styrensulfonsäure-co-Maleinsäure), sulfatierte Glykosaminoglykane, insbesondere Heparin, selektiv desulfatierte Heparinderivate, Heparansulfat, Chondroitinsulftat, Keratansulfat und Dermatansulfat, ausgewählt sein. Die ungeladenen Bausteine können Aminogruppen oder Thiolgruppen enthaltende Polymere oder Vernetzermoleküle mit mindestens zwei Aminogruppen oder Thiolgruppen sein, wobei die geladenen und ungeladenen Bausteine zu dem Polymernetzwerk vernetzt sind, erhältlich durch eine Aktivierung von Carboxylgruppen der Poly(Acrylsäure-co-4-acrylamidomethylbenzensulfonsäure) und/oder der Poly(Acrylsäure-co-Acrylamidoethansulfonsäure) und/oder der Poly(Acrylsäure-co-Acrylamidoethanhydrogensulfat) und/oder Poly(4-Styrensulfonsäure-co-Maleinsäure), und/oder sulfatierten Glykosaminoglykanen, insbesondere Heparin, und/oder selektiv desulfatierten Heparinderivaten und/oder Heparansulfat, und/oder Chondroitinsulfat und/oder Keratansulfat und/oder Dermatansulfat mit EDC/Sulfo-NHS und entweder eine direkte Vernetzung mit den Aminogruppen enthaltenden Polymeren oder den Vernetzermolekülen mit den mindestens zwei Aminogruppen jeweils unter Amidbildung oder eine Funktionalisierung der aktivierten Carboxylgruppen mittels bifunktionellen Vernetzermolekülen, welche jeweils eine Aminogruppe und eine zu einer Michael-Typ-Addition fähige Gruppe enthalten, und die anschließende Vernetzung mit den Thiolgruppen enthaltenden Polymeren oder den Vernetzermolekülen mit den mindestens zwei Thiolgruppen jeweils über eine Michael-Typ-Addition.

Die elektrische Impedanz des erfindungsgemäßen Hydrogelmaterials ist, gemessen bei einer Frequenz von 0,01 Hz, im Bereich von 150 $\Omega$ bis 10 $\Omega$ variierbar. Vorzugsweise werden die Parameterwerte einer Parameterkonfiguration so gewählt, dass eine elektrische Impedanz von vorzugsweise 30 $\Omega$ erreicht ist. Der bevorzugte Impedanzwert von 30 $\Omega$ wird erreicht, wenn eine Parameterkonfiguration mit den Parametern P1, P2, P3 für P1 =110 $\mu$mol/ml, für P2=4,5 mmol/(g/mol) und für P3=-5,9 A$^{-2}$ vorgegeben wird. Ein weiterer vorteilhafter Impedanzwert von 63 $\Omega$ wird erreicht, wenn eine Parameterkonfiguration mit den Parametern P1, P2, P3 für P1=12 $\mu$mol/ml , für P2=4,5 mmol/(g/mol) und für P3=6,8 A$^{-2}$ vorgegeben wird.

Die elektrische Ladungsspeicherkapazität des erfindungsgemäßen Hydrogelmaterials ist im Bereich von 900 mC/ml bis 4000 mC/ml variierbar. Vorzugsweise werden die Parameterwerte einer Parameterkonfiguration so gewählt, dass eine Ladungsspeicherkapazität von 3040 mC/ml erreicht ist. Die bevorzugte Ladungsspeicherkapazität von 2480 mC/ml wird erreicht, wenn eine Parameterkonfiguration mit den Parametern P1, P2, P3 für P1 =110 $\mu$mol/ml, für P2=4,5 mmol/(g/mol) und für P3=-5,9 A$^{-2}$ vorgegeben wird. Eine weitere vorteilhafte Ladungsspeicherkapazität von ebenfalls 3040 mC/ml wird erreicht, wenn eine Parameterkonfiguration mit den Parametern P1, P2, P3 für P1 =12 µmol/ml, für P2=4,5 mmol/(g/mol) und für P3=6,8 A$^{-2}$ vorgegeben wird.

Die elektrisch leitfähige Komponente kann ein Π-konjugiertes, elektrisch leitfähiges Polymer oder eine Polymerzusammensetzung aus Polypyrrol, Polyanilin, Polythiophen und/oder Poly(3,4-ethylendoxythiophen) (PEDOT) sein.

Gemäß einer Weiterbildung des erfindungsgemäßen leitfähigen Hydrogels kann vorgesehen sein, dass die Aminogruppen und Thiolgruppen enthaltenden Polymere als ungeladene Bausteine ausgewählt sind aus der Klasse der Polyethylenglykole (PEG), Poly(2-oxazoline) (POX), Polyvinylpyrrolidone (PVP), Polyvinylalkohole (PVA) und/oder Polyarylamide (PAM) und dass die Aminogruppen oder Thiolgruppen enthaltenden Vernetzermoleküle nichtpolymere, bifunktionelle Vernetzermoleküle sind.

Weiter kann vorgesehen sein, dass als ungeladene Bausteine Polymere mit konjugierten enzymatisch spaltbaren Peptiden, die entweder Lysin oder Cystein als reaktive Aminosäure in der Peptidsequenz aufweisen, für die Polymernetzwerkbildung genutzt werden. Die enzymatisch spaltbaren Peptide können durch humane oder bakterielle Proteasen, insbesondere MMPs, Cathepsine, Elastasen, Aureolysin und/oder Blutgerinnungsenzyme, spaltbar sein.

Gemäß einer weiteren vorteilhaften Weiterbildung des erfindungsgemäßen Hydrogelmaterials kann vorgesehen sein, dass bioaktive und/oder antiadhäsive Moleküle mit einer Aminogruppe oder Carboxylgruppe und/oder zellinstruktive Peptide über Lysin oder Cystein in einer Sequenz an den geladenen Bausteinen Poly(Acrylsäure-co-4-acrylamidomethylbenzensulfonsäure) und/oder Poly(Acrylsäure-co-Acrylamidoethansulfonsäure) und/oder Poly(Acrylsäure-co-Acrylamidoethanhydrogensulfat) und/oder Poly(4-Styrensulfonsäure-co-Maleinsäure), und/oder sulfatierten Glykosaminoglykanen wie Heparin und/oder selektiv desulfatierten Heparinderivaten und/oder Heparansulfat und/oder Chondroitinsulfat und/oder Keratansulfat und/oder Dermatansulfat oder an deren Derivaten mit zur Michael-Typ-Addition fähigen Gruppen unter Ausbildung einer kovalenten Bindung an das Hydrogelnetzwerk angebunden sind. Die bioaktiven Moleküle können antimikrobielle Substanzen, zum Beispiel Antibiotika oder Antiseptika, oder pharmazeutische Wirkstoffe sein.

Vorzugsweise wird das elektrisch leitfähige Hydrogelmaterial mit einer Parameterkonfiguration mit den Paramatern P0, P2, P3 oder mit den Parametern P1, P2, P3 vorgegeben. Die Parameterwerte können in vorgegeben Parameterwertebereichen variiert werden. Der Wert für den Parameter P0 kann in einem Bereich von 0 bis 80 µmol/ml , der Wert für den Parameter P1 in einem Bereich von 0 bis 150 µmol/ml , der Wert für den Parameter P2 in einem Bereich von 0 bis 10 mmol/(g/mol) und der Wert für den Parameter P3 in einem Bereich von $-7 \times 10^{-3}$ bis $7 \times 10^{-3}$ A$^{-2}$ vorgegeben werden.

Das erfindungsgemäße Hydrogelmaterial kann vorzugsweise ein Speichermodul von 0,2 kPa bis 22 kPa aufweisen.

Das erfindungsgemäße Hydrogelmaterial kann zum *in vivo* Faktormanagement zur Kontrolle der Angiogenese, bei Immunerkrankungen, Krebserkrankungen, Diabetis, neurodegenerativen Erkrankungen, Morbus Crohn, Colitis Ulcerosa, Multiple Sklerose, Asthma, Rheumatoide Arthritis oder der kutanen Wundheilung und der Knochenregeneration verwendet werden. Eine weitere Verwendung oder Anwendung des elektrisch leitfähigen Hydrogelmaterials besteht in der elektrischen Stimulation von Zellen oder Geweben.

Weiterhin kann das erfindungsgemäße Hydrogelmaterial zur gezielten Aufreinigung von Proteinen aus Zelllysaten mikrobieller oder eukaryotischer Herkunft verwendet werden.

Eine Verwendung des erfindungsgemäßen Hydrogelmaterials kann außerdem zur *in vitro* Zell- und Organkultur von induziert pluripotenten Stammzellen (iPS-), sowie weiteren, nicht iPS zuzuordnenden Stammzellen und Vorläuferzellen, primären, von Patienten gewonnen Zellen, immortalisierten Zelllinien, sowie Herzgewebe, Muskelgewebe, Nierengewebe, Lebergewebe und Nervengewebe vorgesehen sein.

Kern der Erfindung ist die Bereitstellung des erfindungsgemäßen Hydrogels, welches als wichtigstes Merkmal bei physiologischen Bedingungen (Ionenstärke und pH-Wert) anionische, das heißt negativ geladene Gruppen trägt. Die physikochemischen Eigenschaften des erfindungsgemäßen Hydrogelmaterials, insbesondere die für die intrinsische Affinität zu Substanzen entscheidende (anionische) Ladungscharakteristik und die Charakteristik der für nicht-ionische Wechselwirkungen entscheidenden chemischen Umgebung der anionischen Gruppen sind dabei über folgende Parameter P0, P1, P2 und P3 beschrieben. Zusätzlich zu den bereits beschriebenen Eigenschaften können durch den Einfluss eines elektrischen Potentials auch physikalische Eigenschaften wie Quellung, Steifigkeit und Maschenweite des erfindungsgemäßen Hydrogelmaterials verändert beziehungsweise beeinflusst werden. Die Gegenwart von kovalent gebunden Signaleinheiten ist dabei über weite Bereiche und weitgehend unabhängig von den Parametern P0-P3 abstufbar.

Zur Herstellung des erfindungsgemäßen Hydrogelmaterials wird ein vorgegebenes natives Polymernetzwerk, welches eine vorgegebene Parameterkonfiguration mit vorgegebenen Parameterwerten aufweist, in einer Lösung, in welcher die elektrisch leitfähige Komponente enthalten ist, gequollen und anschließend die Polymerisation oder Vernetzung zu einem elektrisch leitfähigen Polymernetzwerk eingeleitet. Das native Polymernetzwerk mit seinen anionischen Gruppen wirkt dabei über elektrostatische Wechselwirkungen dotierend auf die leitfähige Komponente. Das hergestellte Hydrogelmaterial besteht erfindungsgemäß aus einem oder mehreren leitfähigen Polymersystemen, die durch physikalische Wechselwirkungen mit dem vorgegebenen nativen Polymernetzwerk interagieren. Überraschenderweise hat sich gezeigt, dass die Struktur und Verteilung der elektrisch leitfähigen Komponente durch die Parameter P0-P3 und damit einhergehend die elektrischen Eigenschaften elektrischer Widerstand, elektrische Leitfähigkeit und elektrische Ladungsspeicherkapazität und auch die mechanischen und physikochemischen Eigenschaften des Hydrogelmaterials beeinflussbar sind. Durch den Einfluss eines elektrischen Potentials, das heißt über das Anlegen einer Spannung an die elektrisch leitfähige Komponente, sind zudem die Ladungseigenschaften des Polymernetzwerks und somit die Affinität zu bioaktiven Substanzen stufenlos, reversibel und in Echtzeit modulierbar.

Weitere vorteilhafte Eigenschaften ergeben sich aus den überraschenden Einflüssen durch eine Elektrostimulation von Zellen mit Hilfe des erfindunsgemäßen Hydrogelmaterials in Kombination mit der Freisetzung oder der Sequestrierung von bioaktiven Substanzen. Weiterhin können über die Modulation des Stromflusses auch in Abhängigkeit der Parameter P0 bis P3 die mechanischen Eigenschaften des Materials in Echtzeit variiert werden.

Nachstehend wird die Herstellung des erfindungsgemäßen Hydrogelmaterials anhand eines Ausführungsbeispiels näher erläutert:
Zur elektrischen Funktionalisierung des Hydrogelmaterials wird das leitfähige Polymer Poly-3,4-ethylendioxythiophen (PEDOT) als elektrisch leitfähige Komponente um das vorgegebene native Polymernetzwerk polymerisiert. Dazu wird das vollständig gequollene Polymernetzwerk zunächst für 3 Stunden bei Raumtemperatur in einer Lösung bestehend aus 0,4 M Ammoniumperoxodisulfat (APS) gelöst in 1 M HCl inkubiert. Anschließend erfolgt eine Inkubation für 6 Stunden bei Raumtemperatur in 0,4 M 3,4-Ethylendioxythiophen (EDOT) in Mineralöl. Während des zweiten Inkubationsschritts erfolgt eine oxidative Polymerisation von PEDOT durch das native Polymernetzwerk. Erhalten wird ein SPH-PEDOT, welches anschließend in Mineralöl, Hexan und PBS gewaschen wird. Durch die verwendete Methode sind im Vergleich zur Elektrodeposition von PEDOT keine Elektroden notwendig. Zusätzlich können beliebige Volumenkörper elektrisch leitfähig gemacht werden.

Direkt nach der oxidativen Polymerisation besitzt PEDOT eine inhärente positive Ladung (siehe 10.1021/acs.jpcb.9b01745, 10.1021/acsapm.8b00061). Durch die nicht-kovalenten Wechselwirkungen zwischen PEDOT und den anionisch geladenen Bausteinen des Hydrogels sowie der nicht-kovalenten Wechselwirkung zwischen den einzelnen PEDOT-Ketten (wahrscheinlich zumeist hydrophobe Wechselwirkungen) bildet sich ein pseudo-interpenetrierendes Netzwerk zwischen dem anionisch geladenen Polymernetzwerk und PEDOT aus. Gleichzeitig fungieren die negativen Ladungen des anionisch geladenen Polymernetzwerks als Dotierung für PEDOT. In Abhängigkeit der integralen und lokalen Ladungsdichte, welche durch die Parameter P0 oder P1 und P2 definiert sind, ändert sich der Dotierungsgrad des PEDOT. Dies hat einen direkten Einfluss auf die elektrischen Eigenschaften des leitfähigen Hydrogelmaterials. Da es sich bei PEDOT um ein hydrophobes Polymer handelt, ist die Verteilung und die Vernetzung der PEDOT-Ketten untereinander stark von der Hydrophobizität der Umgebung, welche durch den Parameter P3 definiert ist, abhängig, was ebenfalls einen Einfluss auf die elektrischen Eigenschaften des Hydrogelmaterials hat.

Neben einer Freigabe von geladenen Molekülen lassen sich durch die Veränderungen im Dotierungsgrad infolge von vorgegebenen Werten der P1 und P2 sowie der Hydrophobizität P3 leitfähige Hydrogele mit unterschiedlichen elektrischen Eigenschaften erhalten. Durch eine hohe Dotierung können Hydrogelmaterialien mit hoher Leitfähigkeit erhalten werden. Diese können als biokompatible Elektroden verwendet werden. Aufgrund der mechanischen Eigenschaften von Hydrogelmaterialien, welche biologischem Gewebe sehr ähnlich sind, können Fremdkörperreaktionen eines Organismus gegenüber SPH-PEDOT Elektroden im Vergleich zu klassischen Metallelektroden reduziert werden. Zusätzlich findet bei klassischen Metallelektroden der Ladungstransfer für die elektrische Stimulation nur an der Kontaktfläche vom Metall zum Gewebe (physiologische Lösung) statt. Aufgrund der Verteilung der leitfähigen Komponente innerhalb des Hydrogelmaterials und der Möglichkeit, dass physiologische Flüssigkeiten in das Hydrogelmaterial diffundieren können, kann eine Ladungsübertragung innerhalb des Volumens des Hydrogelmaterials stattfinden. Vorteilhaft sind dadurch für die gleiche Ladungsinjektion geringere elektrische Spannungen erforderlich, was eine geringere Wärmeentwicklung und demzufolge Gewebeschonung zur Folge hat.

Das erfindungsgemäße Hydrogelmaterial ermöglicht in Kombination mit dem erfindungsgemäßen Verfahren eine retardierte Freisetzung von Wirkstoffen in ein Biofluid oder Lebendgewebe.

Weitere Einzelheiten, Merkmale und Vorteile von Ausgestaltungen der Erfindung ergeben sich aus der nachfolgenden Beschreibung von Ausführungsbeispielen mit Bezugnahme auf die zugehörigen Zeichnungen und Tabellen. Es zeigen:

Figur 1: eine schematische Darstellung zur Erläuterung des erfindungsgemäßen Hydrogelmaterials,
Figur 2: eine weitere schematische Darstellung zur weiteren Erläuterung der Erfindung,
Figur 3: eine Abbildung, die die optische Erscheinung der elektrisch leitfähigen porösen und nicht porösen Hydrogelmaterialien im Querschnitt zeigt,

Anionisch geladene Hydrogelbausteine sind nachfolgend mit GB abgekürzt, wobei unterschiedliche anionisch geladene Bausteine zusätzlich mit einer Ziffer gekennzeichnet sind. Ungeladene Bausteine des Hydrogelmaterials sind mit UGB abgekürzt, wobei unterschiedliche ungeladene Bausteine mit einer Ziffer gekennzeichnet sind. Der Einfachheit halber und aus Platzgründen sind Hydrogelmaterialien in den Tabellen als Hydrogele bezeichnet. Hydrogelmaterialtypen sind in den Tabellen als Hydrogeltypen bezeichnet.

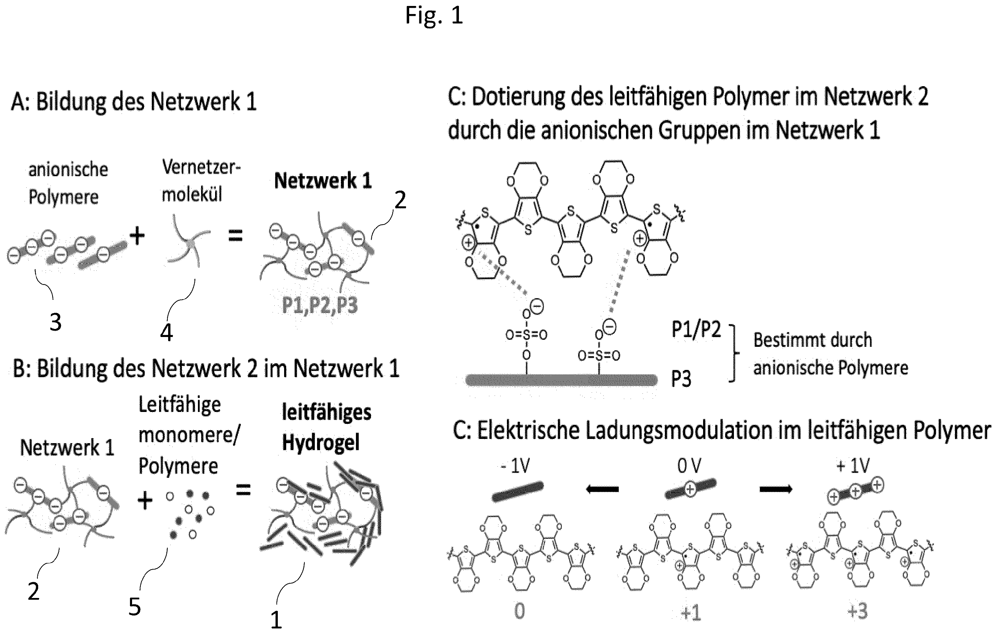

Die **Figur 1** zeigt eine schematische Darstellung zur Erläuterung des erfindungsgemäßen elektrisch leitfähigen Hydrogelmaterials 1. Abbildung A der Figur 1 zeigt beispielhaft die Synthese des Polymernetzwerks 2 aus anionisch geladenen Bausteinen 3 und Vernetzermolekülen 4 als Vorlage zur Bildung des erfindungsgemäßen Hydrogelmaterials 1. Mit dem Polymernetzwerk 2 wird die Parameterkonfiguration mit den Parametern P1, P2 und P3 vorgegeben. Die Abbildung B zeigt den Aufbau des elektrisch leitfähigen Hydrogelmaterials 1 als Pseudo-interpenetrierendes Polymernetzwerk (IPN) aus dem Polymernetzwerk 2 und einer elektrisch leitfähigen Komponente 5. Das Polymernetzwerk 2 trägt anionische Gruppen und wird durch die Parameter P1, P2, und P3 bestimmt. Das elektrisch leitfähige Hydrogelmaterial 1 entsteht durch Polymerisation oder Vernetzung des Polymernetzwerks 2 mit der elektrisch leitfähigen Komponente 5. In der Abbildung C der Figur 1 ist die Dotierung der elekrtisch leitfähigen Komponente 5 beispielhaft gezeigt. Im Beispiel ist die elektrisch leitfähige Komponente 5 PEDOT: Die Dotierung erfolgt über die Wechselwirkung mit sulfat-/sulfonat-Gruppen im anionisch geladenen Polymernetzwerk 2. Für die Wechselwirkung mit PEDOT 5 und die dadurch die resultierenden elektrischen Eigenschaften des leitfähigen Hydrogelmaterials 1 sind die integrale Ladungsdichte P1 des Polymernetzwerks 2, die lokale Ladungsdichte P2 und die Hydrophobizität P3 des anionische Gruppen tragenden Polymers im Polymernetzwerk 2 entscheidend. In Abbildung D ist beispielhaft dargestellt, welchen Einfluss das Anlegen eines elektrischen Potentials auf das elektrisch leitfähige Hydrogelmaterial (1) hat. Durch den Einfluss eines elektrischen Potentials kann die positive Ladung des PEDOT (5) stufenlos reguliert werden. Die Regulierung erfolgt dabei von neutral (0) über mittel (+1) bis stark (+3) positiv geladen.

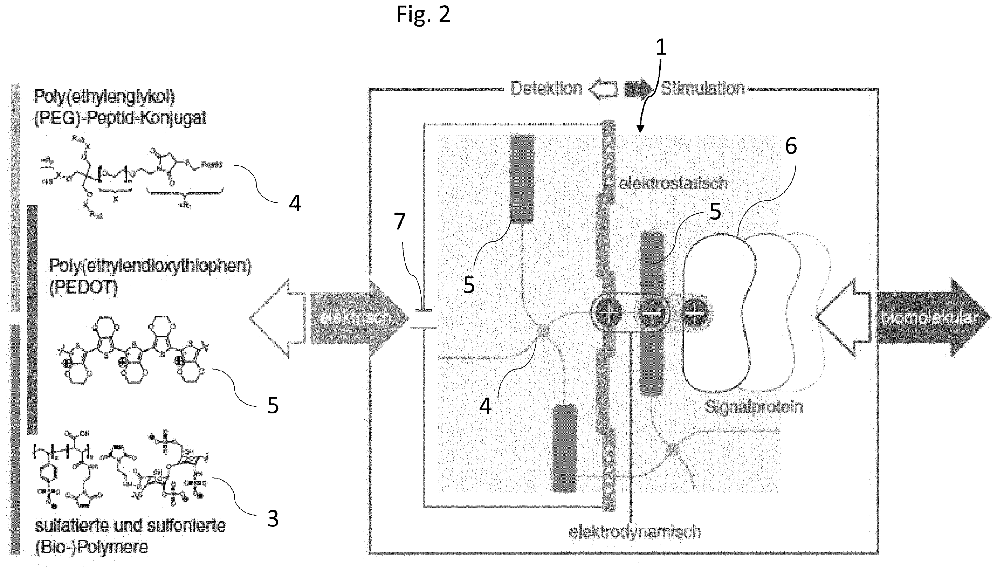

Die **Figur 2** zeigt eine weitere schematische Darstellung zur weiteren Erläuterung der Erfindung. Dargestellt ist ein elektrisch leitfähiges Hydrogelmaterial 1, welches aus sulfatierten und sulfonierten Polymeren als anionisch geladende Bausteine 3 und PEG als Vernetzermoleküle 4, welche ein Polymernetzwerk 2 bilden, und PEDOT als elektrisch leitfähige Komponente 5, welche in dem Polymernetzwerk 2 inkorporiert ist, ausgebildet ist. Rechts mit dem Bezugszeichen 6 ist ein positiv geladenes Signalprotein dargestellt, welches an negativ geladenem PEDOT 5 gebunden ist. Die Bindungseigenschaften des Signalproteins 6 in dem Hydrogelmaterial 1 ist durch den Einfluss eines elektrischen Potentials beeinflussbar. Zur elektrischen Beeinflussung des Hydrogelmaterials 1 ist das Hydrogelmaterial 1 mit einer Spannungsquelle 7 elektrisch kontaktiert. Das erfindungsgemäße Hydrogelmaterial 1 ermöglicht eine elektrodynamische Modulation spezifischer elektrostatischer Wechselwirkungen zwischen dem Hydrogelpolymeren und dem Signalprotein 6.

Ausführungsbeispiele:

## Synthese des Polymernetzwerks

Für die Synthese des Hydrogelmaterials 1 wurden drei anionisch geladene Polymernetzwerke 2, (1) ein aus zwei-Hydrogelbausteinen eines anionisch geladenen Bausteins (GB1-5) mit Maleimid- und eines ungeladenen Bausteins (UGB2) mit Thiolgruppen (im weiteren bezeichnet als Maleimid-Thiol Zwei-Komponenten-System) oder (2) ein aus drei Hydrogelbausteinen (ein anionisch geladener Baustein (GB1-5) mit Maleimidgruppen und ein ungeladener Baustein mit Maleimidgruppen (UGB1)) und ein ungeladener Baustein (UGB2) mit Thiolgruppen (im weiteren bezeichnet als Maleimid-Thiol Drei-Komponenten-System) oder (3) ein System aus zwei Hydrogelbausteinen, vernetzt über EDC/NHS-basierte Aktivierung der Carboxylgruppen des anionisch geladenen Hydrogelbausteins (GB1) und Reaktion mit den Aminogruppen am zweiten, ungeladenen Hydrogelbaustein (UGB3) (im weiteren als EDC/NHS-System bezeichnet), verwendet.

Die Eigenschaften der anionisch geladenen Hydrogelbausteine (GB1 bis GB5) und der ungeladenen Hydrogelbausteine (UGB1 bis UGB3) sind **Tabelle 1** zu entnehmen.

## Maleimide-Thiol Zwei-Komponenten-System zur Herstellung eines anionisch geladenen Polymernetzwerks als Vorstufe des erfindungsgemäßen Hydrogelmaterials

GB1 (Heparin-Maleimid, 15 kDa) und UGB2 (sternförmiges Thiolfunktionalisiertes Polythylenglycol, starPEG, 10 kDa) werden in 0,1 x phosphatgepufferter Kochsalzlösung ((PBS), pH = 6) in einer Konzentration von je 0,0015 mol/l gelöst. 1 x PBS besteht aus 137 nm NaCl, 2,7 mM KCl und 12 mM Gesamt-Phosphat, bestehend aus $HPO_4^{2-}$ und $H_2PO_4$. Die Mischungsverhältnisse und Konzentrationen sind der aus der Tabelle 2 zu entnehmen. Alle nachfolgenden Schritte bis zum Mischen der Hydrogelbausteine erfolgen auf Eis. (pH-Werteinstellung zum Erreichen einer Gelierungszeit von 30min) Für ein molares Verhältnis von 1 beider Bausteine werden gleiche Volumen der beiden Lösungen durch Vermischung mittels einer Pipette und/oder in einem Mischgerät gemischt. Anschließend wird die Mischung zentrifugiert, um Luftblasen zu entfernen und die Proben auf Goldelektroden (Durchmesser 11 mm, 100 $\mu$l) oder auf ein Deckglas (Durchmesser 8 mm, 67 µl) pipettiert und mit einem 11 mm oder 8 mm großen hydrophoben (Sigmacote® behandelten) Deckglas abgedeckt. Die Vernetzung des Hydrogels erfolgt über die Reaktion der Thiol- und Maleimidgruppen (Michael-Additionsreaktion). Die Polymerisation erfolgt für mindestens 30 min bei Raumtemperatur in einer feuchten Kammer, um ein Austrocknen der Gele zu vermeiden. Für die Kryogelierung werden die Proben über Nacht bei -15 °C polymerisiert. Anschließend werden die vollständig polymerisierten Hydrogele über Nacht in 1 x PBS (0,154mmol/I NaCl, pH 7,4) gequollen. Der Feststoffanteil der Gele beträgt ca. 3 % (m/v).

### Maleimide-Thiol Drei-Komponenten-System zur Herstellung eines Polymernetzwerks als Vorstufe des erfindungsgemäßen Hydrogelmaterials

Für eine präzise Einstellung der integralen negativen Ladung im Hydrogelmaterial wird ein Drei-Komponenten System verwendet. Es werden starPEG-Thiol, starPEG-Maleimide und Maleimid-funktionalisierte anionisch geladene Hydrogelbausteine in 0,01 x PBS in einer vorgegebenen Konzentration gelöst und in Abhängigkeit der gewünschten integralen Ladung in verschiedenen Verhältnissen gemischt. Die Mischungsverhältnisse sind Tabelle 2 zu entnehmen. Durch Zugabe von 0,01 M HCl beziehungsweise 0,01 M NaOH wird die Zeit zur Gelierung auf weniger als 5 min eingestellt. Anschließend werden die Lösungen gemischt und der Probenkörper analog zum Zwei-Komponenten-System bei Raumtemperatur (30 min) beziehungsweise 15 °C (über Nacht) erzeugt. Die vollständig polymerisierten Hydrogele werden über Nacht in 1 x PBS (pH 7) gequollen. Der Feststoffanteil der Gele beträgt ca. 3 % (m/v).
Die Eigenschaften der ungeladenen und anionisch geladenen Bausteine sind in Tabelle1 gezeigt.

### EDC/NHS-System zur Herstellung eines Polymernetzwerks als Vorstufe des erfindungsgemäßen Hydrogelmaterials

Für das EDC/NHS-System werden für ein molares Verhältnis von UGB3 sternPEG-Amin zu nicht-maleimidierten GB1 von 2 0,167 mg/µl von UGB3, 0,145 mg/µl GB1, 0,1 mg/µl EDC (N-(3-Dimethylaminopropyl)-N'-ethylcarbodiimid,) und 0,1 mg/µl NHS (N-Hydroxysulfosuccinimid) in eiskaltem MilliQ-Wasser gelöst. Für 1 ml finales Gelvolumen werden zunächst 85,20 µl EDC-Lösung und 48,25 µl NHS-Lösung zu 533,22 µl Heparin-Lösung pipettiert, in einem Labormixer gemischt (Vortexen) und für 15 min auf Eis inkubiert. Anschließend erfolgt die Zugabe von 333,33 µl starPEG-Lösung und das erneute Mischen durch Vortexen. Direkt im Anschluss erfolgt das Erzeugen der gewünschten Formkörper identisch zum Zwei-Komponenten-System. Die Polymerisation erfolgt über Nacht bei Raumtemperatur oder -15 °C in einer feuchten Kammer, um das Austrocknen der Hydrogele zu vermeiden. Der Gesamtfeststoffgehalt der Gele beträgt ungefähr 10 % bei einer finalen starPEG-Konzentration von 0,00784 mol/l und einer finalen Heparin-Konzentration von 0,00392 mol/l. Die vollständig polymerisierten Hydrogele wurden über Nacht in 1 x PBS (pH 7) gequollen.

### Synthese des elektrisch leitfähigen Hydrogelmaterials als pseudo-interpenetrierendes Netzwerk

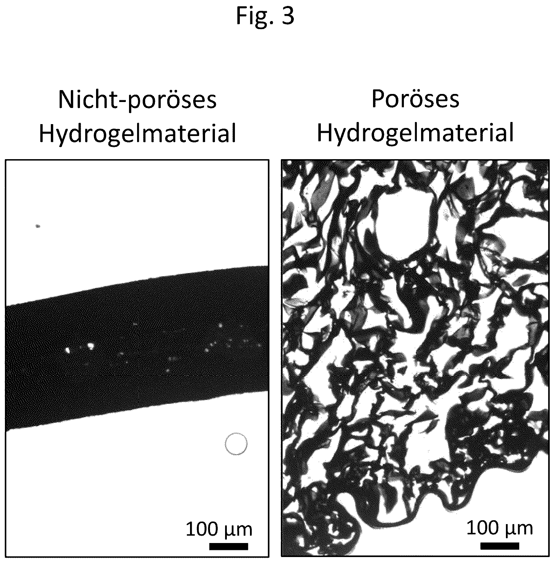

Die Synthese des elektrisch leitfähigen pseudo-interpenetrierenden Polymernetzwerks ist für sämtliche Hydrogelmaterialien der zuvor beschriebenen Bildungsvorschriften möglich. Für die Synthese des elektrisch leitfähigen pseudo-interpenetrierenden Poly-3,4-ethylendioxythiophen (PEDOT)-Netzwerks mit PEDOT als elektrisch leitfähige Komponente, werden die vollständig gequollenen anionisch geladenen Polymernetzwerke im ersten Schritt für 3 Stunden in einer 0,4 M Ammoniumperoxodisulfat (APS) Lösung in 1 M HCl bei Raumtemperatur inkubiert. Anschließend wird das Hydrogel für 6 Stunden in eine 0,4 M 3,4-Ethylendioxythiophen (EDOT) Lösung in Mineralöl in einem Rotationsschüttler inkubiert. In dieser Zeit erfolgt die oxidative Polymerisation des EDOT zu einem elektrisch leitfähigen PEDOT-Netzwerk, welches mit zunehmender Polymerisation des PEDOT eine schwarze Farbe annimmt (Figur 3). Anschließend werden die so hergestellten Hydrogelmaterialien über Nacht in Mineralöl gewaschen, um nicht reagierte Monomere zu entfernen. Zum Entfernen des Mineralöls werden die Hydrogelmaterialien anschließend in Hexan gewaschen. Der Waschvorgang kann wiederholt werden. Anschließend werden die Hydrogelmaterialen für mindestens 24 h in PBS (pH 7,4) gewaschen.
Alle Hydrogelmaterialtypen sind in **Tabelle 2** zusammengefasst. Bei den angegebenen Konzentrationen handelt es sich um die Konzentration in der fertigen Gelmischung. Die Hydrogelmaterialtypen GB1-UGB2 16, GB1-UGB2 18, GB2-UGB2-UGB1 19, GB2-UGB2-UGB1 20, GB3-UGB2-UGB1 21 und GB4-UGB2-UGB1 22 sind Hydrogele, welche nicht mit PEDOT funktionalisiert wurden. Hydrogelmaterialtyp UGB1-UGB2 15 ist ein reines PEG-PEG Hydrogel, welches keine anionischen Ladungen trägt, allerdings PEDOT-funktionalisiert ist.

### Elektrische Charakterisierung

Die elektrische Charakterisierung sämtlicher Hydrogele erfolgte mittels Impedanzspektroskopie beziehungsweise zyklischer Voltammetrie in einem Drei-Elektroden-Setup in 100 ml 1x PBS (pH 7). Für Beide Messmethoden wurden 100 µl Gel-Probenkörper (Volumen ungequollen) auf einer Gold-Netzelektrode hergestellt, welche als Arbeitselektrode dient. Als Gegenelektrode wurde eine poröse Kohlenstoff-Elektrode (BioLogic A-010530) mit deutlich größerer Oberfläche verglichen zur Arbeitselektrode verwendet. Als ReferenzElektrode diente eine Ag/AgCl-Elektrode (Metrohm, Part No. 6.0726.100). Als Messgerät diente ein Potentiostat (Metrohm Autolab PGSTAT204 oder PalmSens 4).

**Impedanzspektroskopie**

Für die Impedanzspektroskopie wird ein Effektivpotential von 10 mV$_{rms}$ angelegt und die Impedanz sowie die der Phasenwinkel in einem Frequenzbereich von 0,01 Hz bis 10$^5$ Hz mit zehn Messpunkten je Dekade gemessen. Die Impedanz der verschiedenen Hydrogelmaterialtypen wurde bei einer Frequenz von 0,01 Hz verglichen. Die elektrische Leitfähigkeit verhält sich umgekehrt proportional zur Impedanz. Steigt die Impedanz so sinkt die elektrische Leitfähigkeit.

**Zyklische Voltammetrie**

Die Berechnung der Ladungsspeicherkapazität erfolgt anhand der Messung der zyklischen Voltammetrie. Dafür wird unter Verwendung des oben genannten Drei-Elektroden-Aufbaus der Stromfluss zwischen Arbeitselektrode (Hydrogel) und Gegenelektrode (Kohlenstoff-Elektrode) gemessen, während in fünf zyklischen Durchläufen das angelegte elektrische Potential von -0,6 bis 0,8 V (Potential zwischen Arbeitselektrode und Ag/AgCl-Referenzelektrode) variiert wird. Die Scan-Rate beträgt 50 mV/s. Anschließend wird der negative Teil der Fläche unter der Kurve integriert (MultiTrace 4.3, PalmSens 4) und daraus die Ladungsspeicherkapazität des Hydrogels mit nachfolgender Formel berechnet:

$$Ladungsspeicherkapazität = \frac{I * U}{Scan - Rate}$$

Als Integral gibt die Software den Wert I * U in der Einheit [A] * [V] aus. Dabei gilt [A] = [C/s]. Durch die Division der Scan-Rate [V/s] resultiert die Ladung [C], welche durch das Material übertragen wird. Diese wird anschließend in das Verhältnis zum Volumen gesetzt, da die Grenzfläche zum umgebenden Medium im kompletten Volumen des Hydrogels vorhanden ist und sich somit keine Oberfläche berechnen lässt. Die Angabe der Werte erfolgt in Ladung pro Milliliter Hydrogel.

**Elektrische Eigenschaften der Hydrogelmaterialien**

Direkt nach der oxidativen Polymerisation besitzt PEDOT eine inhärente positive Ladung (10.1021/acs.jpcb.9b01745, 10.1021/acsapm.8b00061). Durch die nicht-kovalenten Wechselwirkungen zwischen PEDOT und dem anionisch geladenen Polymernetzwerk (Hydrogel) sowie der nicht-kovalenten Wechselwirkung zwischen den einzelnen PEDOT-Polymerketten bildet sich ein pseudo-interpenetrierendes Netzwerk zwischen dem anionisch geladenen Polymernetzwerk und PEDOT aus. Gleichzeitig fungieren die negativen Ladungen des anionisch geladenen Polymernetzwerks als p-Dotierung für PEDOT. In Abhängigkeit der integralen und lokalen Ladungsdichte (Parameter P0/P1 und P2) ändert sich der Dotierungsgrad des PEDOT. Dies hat einen direkten Einfluss auf die elektrischen Eigenschaften (Impedanz und Ladungsspeicherkapazität) des elektrisch leitfähigen Hydrogelmaterials. Bei gleichem P2 und P3 wurden über einen P1-Bereich von 110 bis 2 $\mu$mol/ml eine Ladungsspeicherkapazität von 3040 bis 1505 mC/ml und eine Impedanz von 30 bis 93 $\Omega$ für die Hydrogelmaterialtypen GB1-UGB2 01 und GB1-UGB2-UGB1 02-04 und in einem P1-Bereich von 128 bis 2 $\mu$mol/ml eine Ladungsspeicherkapazität von 3040 bis 1894 mC/ml und eine Impedanz von 36 bis 74 $\Omega$ für die Hydrogelmaterialtypen GB2-UGB2 05 und GB2-UGB2-UGB1 06-08 (**Tabelle 3-1 und 3-2**) erhalten. Da es sich bei PEDOT um ein hydrophobes Polymer handelt, ist die Verteilung und die Vernetzung der PEDOT-Ketten untereinander stark von der Hydrophobizität der Umgebung und damit der Amphiphilie des anionisch geladenen Polymernetzwerks (Parameter P3) abhängig, was ebenfalls einen Einfluss auf die elektrischen Eigenschaften des elektrisch leitfähigen Hydrogelmaterials hat. In den Ausführungsbeispielen zeigt sich, dass bei einem hohen Wert des Parameters P3 von 6,8 *10$^{-3}$ 1/A$^2$ (GB2-UGB2-UGB1 08), siehe Tabelle 2, im Vergleich zu einem niedrigen Wert für P3 von -5,9*10$^{-3}$ 1/A$^2$ (GB1-UGB2-UGB1 04), bei gleich bleibendem Wert für P2 (4,5 mmol/(g/mol)) und P1 (2 $\mu$mol/ml) die höhere Hydrophobizität zu einer höheren Ladungsspeicherkapazität (1894 vs. 1505 mC/ml) und geringerer Impedanz (74 $\Omega$ vs. 93 $\Omega$) führt (Tabelle 3-1 und 3-2). Erhöht man den Abstand zwischen den stark anionisch geladenen Gruppen entlang der Polymerketten (verringerter Wert P2, steht für eine geringere lokale Ladungsdichte) so erhält man bei ähnlichem P3 und annähernd gleichem P1 eine deutlich geringere Ladungsspeicherkapazität bei gestiegener Impedanz. Dieser Unterschied zeigt sich besonders bei einem Vergleich bei niedrigem P1 von 9 mmol/ml, P2 von 0,9 mmol/(g/mol) und P3 von 0,7*10$^{-3}$ 1/A$^2$ (GB4-UGB2-UGB1 12) im Vergleich zu einem P1 von 11 beziehungsweise 4 mmol/ml, P2 von 4,5 mmol/(g/mol) und P3 von -5,9*10$^{-3}$ 1/A$^2$ (GB1-UGB2-UGB1 02/03). Trotz des geringeren Wertes für P3 und einem annähernd gleichen beziehungsweise geringerem Wert für P1 besitzen die Hydrogele mit höherem P2 (GB1-UGB2-UGB1 02/03) eine deutlich höhere Ladungsspeicherkapazität (2350 und 1811 mC/ml und geringere Impedanz (42 und 81 Ω) im Vergleich zu GB4-UGB2-UGB1 12 (910 mC/ml und 150 Ω). Trotz der moderaten Werte für P1 und P3 verhält sich GB4-UGB2-UGB1 12 wie ein Hydrogel ohne anionisch geladene Gruppen nach PEDOT-Funktionalisierung, siehe UGB1-UGB2 15 (reines PEG-Hydrogelmaterial, 921 mC/ml und 167 Ω). Es lassen sich somit durch die Veränderungen im Dotierungsgrad (P1, P2) sowie der Hydrophobizität (P3) leitfähige Hydrogele mit unterschiedlichen elektrischen Eigenschaften erhalten. Durch eine hohe integrale Anzahl an anionischen Gruppen (d.h. einem hohen Wert für P0 bzw. P1) sowie einen geringen Abstand zwischen den stark anionisch geladenen Gruppen (P2) können Hydrogele mit hoher Leitfähigkeit erhalten werden. P0 scheint einen ähnlichen Einfluss wie P1 auf die elektrischen Eigenschaften zu haben, der vermutlich auf den Anteil der stark anionischen Gruppen zurückzuführen ist. Die schwach anionischen Gruppen, hier beispielsweise Carboxylgruppen mit einem intrinsischen pKa im Bereich von 3,5 bis 4,5 spielen vermutlich eine untergeordnete Rolle bei der Dotierung des PEDOT. Durch den Parameter P3 lässt sich ebenfalls die Interaktion zwischen hydrophilem/amphiphilem anionischem Polymer und hydrophobem elektrisch leitfähigem Polymer (PEDOT) konfigurieren. Durch eine erhöhte Hydrophobizität der anionisch geladenen Hydrogelbausteine (hoher Wert für Parameter P3) lassen sich bereits bei deutlich geringerem P1 gesteigerte Leitfähigkeiten (d.h. geringere Impedanzen) erzeugen. Dies liegt an der hohen Affinität der hydrophoben PEDOT-Einheiten zu den hydrophoben Gruppen auf den anionischen geladenen Hydrogelbausteinen, welche möglicherweise die Dotierung positiv beeinflussen. Zusätzlich erleichtern die hydrophoben Gruppen bereits während der PEDOT-Synthese die Penetration und Verteilung der Monomereinheiten (EDOT) im anionisch geladenen Polymernetzwerk. Die elektrisch leitfähigen Hydrogelmaterialien mit sehr hoher Leitfähigkeit (geringer Impedanz) und hoher Ladungsspeicherkapazität können als biokompatible Elektrodematerialien zur Stimulation von Zellen oder Gewebe verwendet werden. Aufgrund der hohen Hydratisierung und Weichheit dieser Hydrogele, welche biologischem Gewebe sehr ähnlich sind, können die Fremdkörperreaktionen des Organismus gegenüber solchen auf den erfindungsgemäßen, elektrisch leitfähigen Hydrogelmaterialien basierenden Elektroden im Vergleich zu klassischen Metallelektroden deutlich reduziert werden. Zusätzlich findet bei klassischen Metallelektroden der Ladungstransfer für die elektrische Stimulation nur an der Kontaktfläche vom Metall zum Gewebe (physiologische Lösung) statt. Aufgrund der Verteilung des leitfähigen Polymers innerhalb des Volumenmaterials und der Möglichkeit, dass Biofluide in das Hydrogel eindiffunieren, kann eine Ladungsübertragung innerhalb des kompletten Volumens stattfinden. Dadurch können für die gleiche Ladungsinjektion geringere Spannungen verwendet werden, was die Wärmeentwicklung und daraus resultierende potentielle Gewebeschäden im Vergleich zu klassischen Metallelektroden reduziert.

## Wirkstoffsequestrierung und -freisetzung

Die Probenvorbereitung für die Wirkstoffsequestrierung und -freisetzung erfolgt identisch zur Charakterisierung der elektrischen Eigenschaften. 100 µl der verschiedenen anionisch geladenen Polymernetzwerke wurden auf eine Gold-Netzelektrode aufgebracht und mittels nachfolgender Penetration und Polymerisation von EDOT-Monomeren zu PEDOT elektrisch leitfähig gemacht.

## Wirkstoffsequestrierung

Nach gründlichem Waschen der elektrisch leitfähigen Hydrogelmaterialien in PBS erfolgte die Sequestrierung verschiedener Substanzen mit einer Konzentration von jeweils 100 ng/ml Substanz in 2,5 ml PBS mit 0,1% BSA (um die physiologische Situation mit einem Trägerprotein nachzustellen. Das entspricht 250 ng pro Protein und Hydrogelmaterial. Die Sequestrierung erfolgt in einem 5 ml *low binding* Eppendorf Tube (Eppendorf) um unspezifische Bindung der Proteine zum Reaktionsgefäß zu minimieren. Die Aufnahme erfolgt über 24 h.

Die Sequestrierung erfolgt bei 500 mV, 0 mV (passiv) und -500 mV. Die aktive Sequestrierung erfolgt in einem Drei-Elektroden-Setup ähnlich der elektrischen Charakterisierung. Als Arbeitselektrode diente das leitfähige Hydrogel auf der Gold-Elektrode. Diese befand sich zusammen mit der Referenzelektrode, einem Ag/AgCl Draht, in einem 5 ml *low binding* Eppendorf Tube. Die Gegenelektrode, eine poröse Kohlenstoffelektrode (BioLogic A-010530) mit deutlich größerer Oberfläche verglichen zur Arbeitselektrode, befand sich in einem separaten Gefäß mit 100 ml PBS. Geschlossen wurde der Stromkreis durch eine Salzbrücke, bestehend aus einem PVC-Schlauch, welcher mit einem 25%igen Polyacrylamidhydrogel (nach Herstellerangaben angeben) gequollen in PBS gefüllt war. Im Eppendorf-Gefäß war der Schlauch zusätzlich mit einer 1000 Da Dialysemembran verschlossen, um ein Eindringen der Substanzen zu verhindern. Für das Anlegen eines definierten Potentials diente ein Potentiostat (Metrohm Autolab PGSTAT204 oder PalmSens 4). Als Proben wurden 100 µl der Lösung vor und nach der Sequestrierung entnommen. Nach Bestimmung der Konzentrationen der verschiedenen Proteine nach Herstellerangaben unter Verwendung des Multiplex Assays Kits (Luminex Technology, ThermoFisher) wurde die aufgenommene Menge an Protein prozentual berechnet.

Für die Sequestrierung von Substanzen spielen vorwiegend die integrale P0 beziehungsweise P1 und lokale Ladungsdichte P2 bei den verschiedenen angelegten Spannungen im gesamten Hydrogelmaterial (anionisch geladenes Polymernetzwerk -PEDOT pseudo IPN) eine Rolle. Die höchste Sequestrierung konnte dabei, unabhängig von der Ladung des gebundenen Moleküls, ohne angelegtes Potential gemessen werden (**Tabelle 4-1, 4-2, 4-3 und 4-4**). Im Vergleich zu nicht leitfähigen Hydrogelen (ohne PEDOT) mit ähnlichen P1 und P2 zeigt sich, dass die elektrisch leitfähigen Hydrogelmaterialien eine geringere Aufnahme positiv geladener Substanzen (GB2-UGB2-UGB1 09; 72,3 % SDF-$1\alpha$; 72,7 % FGF-2; 70,8 % IL-8 gegenüber GB2-UGB2-UGB1 19; 98,2 % SDF-$1\alpha$; 85,0 % FGF-2; 96,7 % IL-8) aufweisen (siehe Tabelle 4-1, 4-2, 4-3 und 4-4). Bei negativ geladenen Molekülen zeigt sich ein umgekehrter Effekt. Eine Aufnahme von 49,9 % GM-CSF und 37,1 % EGF bei GB2-UGB2-UGB1 09 im Vergleich zu 43,3 % GM-CSF und 33,5 % EGF bei GB2-UGB2-UGB1 19 konnten gemessen werden. Dieser Effekt ist noch stärker ausgeprägt bei einem geringerem P1. So konnten 77,8 % GM-CSF und 69,2 % EGF bei GB2-UGB2-UGB1 10 im Vergleich zu 0,0 % GM-CSF und 21,0 % EGF bei GB2-UGB2-UGB1 20 gebunden werden. Grund dafür ist wahrscheinlich die positive Ladung des PEDOT. PEDOT weist ohne Anlegen eines elektrischen Potentials eine positive Ladung von ca. 33 % der Monomereinheiten auf, welche mit negativ geladen Substanzen eine Wechselwirkung verursachen und diese wahrscheinlich binden können (10.1021/acsapm.8b00061, 10.1021/acsami.5b04768). Zusätzlich zeigt sich, dass bei einem niedrigen P1 auch positiv geladene Substanzen durch die elektrisch leitfähigen Hydrogele besser im Vergleich zur nicht leitfähigen Kontrolle gebunden werden konnten. So konnten bei GB2-UGB2-UGB1 10, 76,9 % SDF-$1\alpha$; 86,1 % FGF-2; 39,9 % IL-8, gegenüber GB2-UGB2-UGB1 20, 60,5 % SDF-$1\alpha$; 41,6 % FGF-2; 17,1 % IL-8, gebunden werden. Dies lässt vermuten, dass auch für die Bindung vorwiegend positiv geladener Substanzen das leitfähige Polymer PEDOT vor allem durch wechselseitige Ladungskompensation mit den anionisch geladenen Gruppen des Hydrogelmaterials eine wichtige Rolle spielt. Sowohl die ionische Bindung negativ geladener Domänen im Protein durch PEDOT als auch hydrophobe Wechselwirkungen zwischen Protein und dem PEDOT spielen hier eine wichtige Rolle.

Wird ein positives Potential von 500 mV angelegt, verringert sich insgesamt die Sequestrierung von Substanzen (Tabelle 4-1, 4-2, 4-3 und 4-4). Die ist vor allem bei positiv geladenen Substanzen zu beobachten. Bei negativ geladenen Substanzen, wie beispielsweise GM-CSF und EGF, lässt sich hingegen eine geringe Steigerung beziehungsweise eine nahezu identische Sequestrierung beobachten. So konnten 49,9 % GM-CSF und 37,1 % EGF vor Anlegen des Potentials und 59,2 % GM-CSF und 67,0 % EGF nach Anlegen des Potentials bei GB2-UGB2-UGB1 09 beziehungsweise 77,8 % GM-CSF und 69,2 % EGF vor Anlegen des Potentials und 78,4 % GM-CSF und 64,8 % EGF nach Anlegen des Potentials bei GB2-UGB2-UGB1 09 gebunden werden. Grund dafür kann sein, dass sich durch das Anlegen eines positiven Potentials die Ladung des PEDOT erhöht. (10.1021/acsapm.8b00061, 10.1021/acsami.5b04768). Dies führt zu einer positiven Beeinflussung der Bindung negativ geladener Proteine wie GM-CSF und EGF durch die erhöhte ionische Wechselwirkung. Da die negative Ladung der anionischen Komponente im Hydrogel hingegen konstant ist und die stärkere positive Ladung des PEDOT zur teilweisen Ladungskompensation führen kann, verringert sich summarisch die Aufnahme positiv geladener Proteine.

Wird ein negatives Potential angelegt, kann die positive Ladung des PEDOT reduziert beziehungsweise sogar komplett neutralisiert werden (10.1021/acsapm.8b00061, 10.1021/acsami.5b04768). Bei einem Potential von -500 mV zeigt sich vorwiegend eine Reduzierung der Substanz-Sequestrierung unabhängig von der Ladung der Substanzen und unabhängig vom P1 und P2 der Hydrogelmaterialien (**Tabelle 5-1, 5-2, 5-3 und 5-4** ). So sinkt die Sequestrierung von SDF-$1\alpha$ von 72,3 % auf 47,2 %, NGF-$\beta$ von 94,1 % auf 75,0 % und IL-8 von 70,8 % auf 35,4 % in GB2-UGB2-UGB1 09. Ein gleicher Trend lässt sich für GB2-UGB2-UGB1 10 und GB3-UGB2-UGB1 11/12 beobachten. Dies bestätigt die Vermutung, dass für die Bindung vorwiegend positiv geladener Substanzen die positive Ladung des PEDOT eine wichtige Rolle spielt. Dies kann z.B. durch die Bindung negativ geladener Domänen der Substanzen (Proteine) an die positiven Ladungen von PEDOT, das zuvor beschriebene Phänom, verursacht werden. Bei der Sequestrierung negativ geladener Substanzen zeigt sich bei -500 mV eine sehr starke Reduzierung der Sequestrierung. So sinkt die Sequestrierung von GM-CSF von 49,9 % auf 17,9 % und EGF von 37,1 % auf 0,0 % in GB2-UGB2-UGB1 09 beziehungsweise von GM-CSF von 77,8 % auf 44,9 % und EGF von 69,2 % auf 42,8 % in GB2-UGB2-UGB1 10. Der Grund für diese starke Verringerung der Sequestrierung von negativ geladenen Substanzen bei negativem Potential liegt vermutlich in der Reduzierung der positiven Ladung des PEDOT. Dadurch finden sich deutlich weniger Bindungsstellen für negativ geladene Substanzen.

## Wirkstofffreisetzung

Nach der Sequestrierung (Beladung der Hydrogele nach der zuvor angesprochen dritten Beladungsmethode) erfolgte die elektrisch gesteuerte Freisetzung der bioaktiven Moleküle. Aufgrund der im Durchschnitt höchsten Sequestrierung wurden dafür die Proben verwendet, welche bei einem Potential von 0 mV (passiv) für 24 h mit Substanzen beladen wurden. Eine kontrollierte Freisetzung von aktiv sequestrierten Substanzen kann ebenfalls nach dem im folgenden beschriebenen Protokoll durchgeführt werden.

Nach der Beladung der Hydrogele erfolgte zunächst ein kurzer Waschschritt, um schwach gebundene Proteine zu entfernen. Dafür wird das Hydrogel zunächst bei 3000 rpm für 1 min zentrifugiert, um anhaftende Flüssigkeit zu entfernen. Anschließend werden die Hydrogele in 1 ml PBS mit BSA gewaschen und erneut bei 3000 rpm für 1 min zentrifugiert. Die Freisetzung erfolgt ebenfalls in dem im Kapitel Wirkstoffsequestrierung beschriebenen Drei-Elektroden-Setup bei einem angelegten Potential von 500 mV, 0 mV und -500 mV. Die Probenentnahme erfolgte für die Ausgangslösung (Kontrolle), nach Sequestrierung, nach dem Waschen und für die Freisetzung nach 0 min, 10 min, 30 min, 1 h, 6 h, 8 h und 24 h. Da bereits nach 8 h eine Sättigung der freigesetzten Proteine beobachtet werden konnte, sind die Ergebnisse der im Plateau liegenden Freisetzung nach 8 h gezeigt (Tabelle 5-1, 5-2, 5-3 und 5-4). Die Bestimmung der Konzentration der verschiedenen Substanzen erfolgte nach Herstellerangaben unter Verwendung des Multiplex Assays Kits (Luminex Technology, ThermoFisher).

Die Freisetzung der gebundenen Substanzen lässt sich in Abhängigkeit der konfigurierten Parameter des anionisch geladenen Polymernetzwerks in Kombination mit der Ladung des PEDOT einstellen (Tabelle 5-1, 5-2, 5-3 und 5-4). Bei einem hohem P1 (60 $\mu$mol/ml, GB2-UGB2-UGB1 09) erfolgt lediglich eine sehr geringe Freisetzung von positiv geladenen Substanzen (0,0 % SDF-1$\alpha$; 0,0 % FGF-2; 0,5 % IL-8). Für positiv geladene Substanzen kann hingegen eine moderat geringe Freisetzung beobachtet werden (2,3 % GM-CSF; 9,1 % EGF). Durch die wahrscheinliche Überkompensation der positiven Ladung des PEDOT durch die anionischen Gruppen, kann eine Freigabe von negativ geladenen Substanzen wahrscheinlich deutlich schneller erfolgen als die Freigabe von positiv geladenen Proteinen. Bei niedrigem P1 (2 $\mu$mol/ml, GB2-UGB2-UGB1 10) erfolgt eine gesteigerte Freisetzung der positiv geladenen Substanzen (0,8 % SDF-1$\alpha$; 0,3 % FGF-2; 11,7 % IL-8). Negativ geladene Substanzen hingegen werden weniger freigesetzt verglichen mit einem höheren P1 (0,4 % GM-CSF; 2,0 % EGF). Verringert man P2 bei ähnlichen P1 Werten (GB3-UGB2-UGB1 11/12) so kann eine gesteigerte Freisetzung negativ geladener Substanzen (4,3/3,7 % GM-CSF; 94,1/73,5% EGF) bei nahezu unveränderter Freisetzung von positiv geladenen Substanzen erreicht werden (1,6/0,2% SDF-1$\alpha$; 0,0/0,0% FGF-2; 1,4/1,2% IL-8), (Figur 8 Tabelle 5). Grund dafür ist ebenfalls die Kompensation positiver Ladungen im PEDOT durch anionische Gruppen trotz der geringeren Ladungsdichte.

Legt man ein Potential von 500 mV an, so kann ein Zurückhalten gebundener Substanzen unabhängig von deren Ladungen beobachtet werden. Der Effekt zeigt sich besonders deutlich für GB2-UGB2-UGB1 09. Hier konnte die Freisetzung von nahezu allen 14 Proteinen auf 0 % reduziert werden (Tabelle 5-1, 5-2, 5-3 und 5-4). Auch bei moderatem P1 (9 $\mu$mol/ml) und sehr geringem P2 (0,94 mmol/(g/mol)) in GB3-UGB2-UGB1 12 konnte die Freisetzung sämtlicher Substanzen auf nahezu 0 % gesenkt werden (Tabelle 5-1, 5-2, 5-3 und 5-4). Grund dafür könnte in der Quellrate der Hydrogele liegen. Bei einem positiven Potential kompensiert die erhöhte positive Ladung des PEDOT mehr anionisch geladene Gruppen. Geht man davon aus, dass mehr anionische Gruppen verglichen zu den positiven Ladungen des PEDOT im Hydrogelmaterial vorhanden sind, verschiebt sich die gesamte Ladung des Hydrogels hin zum Neutralen, wodurch es zu einem Wasserverlust des Hydrogels und dadurch zu einem Schrumpfen kommt. Dies führt zu einer verringerten Maschenweite der Polymerketten, was wiederum eine reduzierte Freisetzung der Proteine unabhängig von deren Ladung zur Folge hat.

Wird ein Potential von -500 mV angelegt, zeigt sich eine gesteigerte Freisetzung bei einem Großteil der Substanzen (Tabelle 5-1, 5-2, 5-3 und 5-4). Dies betrifft vorwiegend negativ geladene bioaktive Moleküle. So steigt nach Anlegen des Potentials die Freisetzung von TNF-$\alpha$, GM-CSF und EGF für den Hydrogelmaterialtyp GB2-UGB2-UGB1 09 von 1,4/2,3/9,2 % auf 5,3/10,4/67,2 % und GB2-UGB2-UGB1 10 von 1,7/0,4/2,0 % auf 53,2/5,9/72,6 %. Dieser Trend kann auch für die Hydrogelmaterialtypen GB3-UGB2-UGB1 11/12 beobachtet werden. Interessant ist dabei, dass Substanzen, die bereits ohne angelegtes Potential eine größere Freisetzung aufweisen, durch Anlegen des negativen Potentials am meisten freigesetzt werden. Grund dafür ist höchstwahrscheinlich die Verringerung/Neutralisierung der positiven Ladung des PEDOT. Dadurch sind mehr freie anionische Gruppen vorhanden, die durch ionische Wechselwirkungen eine Abstoßung negativ geladener Proteine bewirken. Allerdings kann auch bei positiv geladenen Substanzen eine gesteigerte Freisetzung beobachtet werden. So steigert sich die Freisetzung von FGF-2, IL4 und IL8 für den Hydrogelmaterialtyp GB2-UGB2-UGB1 10 von 0,3 % auf 2,6 %, 0,6 % auf 1,7 % und 11,7 % auf 26,3 %. Grund hierfür könnte ebenfalls die gesteigerte Anzahl an freien anionisch geladenen Gruppen durch fehlende Ladungskompensation mit PEDOT sein. Die dadurch bewirkte erhöhte negative Nettoladung des Hydrogels führt zu einem Quellen. Dadurch wird eine Freisetzung unabhängig von der Ladung begünstigt, wodurch auch positiv geladene Substanzen im höheren Maße freigesetzt werden können.

## Hydrogel Biokompatibilität

Für die Untersuchung der Biokompatibilität wurde die PC12-Zelllinie und der Hydrogelmaterialtyp GB1-UGB2 17 verwendet. Bei der Zelllinie handelt es sich um Phäochromozytom-Zellen, welche durch Zugabe von NGF-$\beta$ innerhalb von wenigen Tagen zu Neuron-artigen Zellen differenziert werden können. Die PC-12-Zelllinie ist ein weit verbreitetes Modell für neuronale Differenzierung. Für die Kultivierung mit PC12 Zellen wurden die gewaschenen und in PBS gequollenen elektrisch leitfähigen Hydrogele zunächst für 10 min in 70 % Ethanol gespült, um eventuelle Mikroorganismen abzutöten und anschließend in sterilem PBS gewaschen. Nach der Behandlung mit Collagen Typ I (50 $\mu$g/ml, 1 ml/100 $\mu$l Gel, Gibco™) in 20 mM steriler Essigsäure für 2 h bei Raumtemperatur wurden die Gele für 30 min in PBS gewaschen. Für NGF-$\beta$ beladene Gele erfolgte eine zusätzliche Inkubation mit NGF-$\beta$ (100 ng/ml, Sigma-Aldrich) in PBS mit 1 % BSA über Nacht. Anschließend wurden 50.000 Zellen pro cm$^2$ Geloberfläche ausgesät. Die Aussaat der Zellen sowie deren Inkubation erfolgte in RPMI1640 (Gibco™) mit einem Zusatz von 10 % Pferdeserum (Gibco™), 5 % fetalem Kälberserum (Gibco™) und 1 % Penicillin und Streptamycin (Gibco™) im Medium. Je nach Kultivierungsbedingung erfolgte die Verwendung des puren Mediums, die Zugabe von 100 ng/ml NGF-$\beta$ in das Medium oder die Verwendung von nährstoffreduziertem Medium (reduziert) mit nur 1 % Pferdeserum (Gibco™) und 0,5 % fetalem Kälberserum. Nach einer Inkubation von 5 beziehungsweise 7 Tagen mit einem Mediumwechsel aller 2 Tage erfolgte das Fixieren der Zellen für 20 min bei Raumtemperatur in 4 % PFA in PBS. Für die Bildgebung wurden die Zellen mit Phalloidin (Abcam) und DAPI (Pierce) nach Herstellerangaben gefärbt. Bilder wurden mit einem Fluoreszenzmikroskop aufgenommen. Die Auswertung der Zellzahl und des Zellumfangs erfolgte mit FIJI/ImageJ unter der Verwendung des *watershed* und *analyze particles* (Zellzahl) und des *skeletonize* Plugins.

Für alle Kultivierungsbedingungen wurden Zelladhäsion und Zellproliferation auf den Gelen beobachtet. Im Vergleich von 7 zu 5 Tagen erfolgte eine starke Zunahme der Zellzahl bei Abnahme des Zellumfangs auf den Hydrogelen ohne Zusatz von NGF-$\beta$ (**Tabelle 6**). Dies spricht für eine hohe Zellproliferation. Die Zugabe von NGF-$\beta$ in das Medium führt zur Differenzierung der PC12-Zellen nach bereits 5 Tagen (Tabelle 6). Dies konnte in einer Zunahme des Zellumfangs durch das Wachstum axonartiger Strukturen bestätigt werden. Eine Zugabe von NGF-$\beta$ in das Hydrogel, nicht aber ins Medium, führt zu einer nur geringen Steigerung des Zellumfangs bei nur gering steigender Zellzahl (Tabelle 6). Ohne Stimulus ist die Menge an freigesetztem NGF-$\beta$ zu gering, um eine vollständige Differenzierung der Zellen zu erreichen. Dies liegt an der hohen Affinität von NGF-$\beta$ zum Hydrogel. Reduziert man den Nährstoffgehalt im Medium, so lässt sich eine gesteigerte Differenzierung erreichen. Die Zellen besitzen demnach in allen Kultivierungsbedingungen die Fähigkeit, auf den Hydrogelen zu proliferieren und differenzieren, was eine hohe Biokompatibilität der Hydrogele bestätigt.

**Bezugszeichenliste**

1    elektrisch leitfähiges Hydrogelmaterial / Hydrogelmaterial
2    Polymernetzwerk
3    anionisch geladene Bausteine
4    Vernetzermolekül
5    elektrisch leitfähige Komponente / PEDOT
6    Signalprotein
7    Spannungsquelle

## Tabelle 1

| Geladene und ungeladene Bausteine | Bau steine Abkürzung | M [g/mol] | Anzahl der Maleimidgruppen [1/mol] | Anzahl der anionischen Gruppen [1/mol] | Anzahl der anionischen Gruppen (pKa<2.5, [1/mol]) | P2 [mmol/ (g/mol)] | P3 $*10^{-3}$ [1/Å$^2$] |
|---|---|---|---|---|---|---|---|
| Heparin | GB1 | 14000 | 7 | 87,6 | 67,6 | 4,5 | -5,9 |
| Poly(4-Styrensulfonsäureco-Maleinsäure) 3:1 | GB2 | 21100 | 9 | 142,8 | 92 | 4,5 | 6,8 |
| Poly(Acrylsäure-co-Acrylamidoethanhydrogensulfat) 1:1 | GB3 | 26200 | 12 | 149 | 80 | 3,2 | -2,5 |
| Poly(Acrylsäure-co-Acrylamidoethanhydrogensulfat) 9:1 | GB4 | 18400 | 12 | 149 | 16 | 0,9 | 0,7 |
| 6-O,N-desulfatiertes Heparin | GB5 | 12300 | 7 | 42,5 | 22,5 | 1,8 | -6,8 |
| 4-Arm-Polyethylenglykol, maleimidterminiert | UGB1 | 10000 | 0 | 0 | 0 | 0 | -2,82 |
| 4-Arm-Polyethylenglykol, thiolterminiert | UGB2 | 10000 | 0 | 0 | 0 | 0 | -2,82 |
| 4-Arm-Polyethylenglykol, amin terminiert thiolterminiert | UGB3 | 10000 | 0 | 0 | 0 | 0 | -2,82 |

**Tabelle 1:** Übersicht über die geladenen und ungeladenen Hydrogelbausteine der Polymernetzwerke

Tabelle 2

| Hydrogelmaterialtyp | geladener Polymerbaustein | Lösungsmittel für geladene und ungeladene Bausteine | GBx (mg/ml) | UGB1 (mg/ml) | UGB2 (mg/ml) |
|---|---|---|---|---|---|
| GB1-UGB2 01 | GB1 | 0,01 x PBS, pH 4 | 30,8 | 0,0 | 21,8 |
| GB1-UGB2-UGB102 | GB1 | 0,01 x PBS, pH 4 | 3,7 | 14,3 | 17,3 |
| GB1-UGB2-UGB1 03 | GB1 | 0,01 x PBS, pH 4 | 1,3 | 16,4 | 16,8 |
| GB1-UGB2-UGB104 | GB1 | 0,01 x PBS, pH 4 | 0,4 | 17,1 | 16,8 |
| GB2-UGB2 05 | GB2 | 0,01 x PBS, pH 4 | 31,8 | 0,0 | 32,1 |
| GB2-UGB2-UGB1 06 | GB2 | 0,01 x PBS, pH 4 | 4,1 | 14,8 | 25,7 |
| GB2-UGB2-UGB1 07 | GB2 | 0,01 x PBS, pH 4 | 1,4 | 16,6 | 16,8 |
| GB2-UGB2-UGB1 08 | GB2 | GB2: 0,01 PBS, pH 4; UGB: 0,01 PBS, pH 5,9 | 0,4 | 17,1 | 16,8 |
| GB2-UGB2-UGB1 09 | GB2 | 0,01 x PBS, pH 4; | 21,2 | 6,9 | 16,8 |
| GB2-UGB2-UGB1 10 | GB2 | 0,01 x PBS, pH 4; | 0,7 | 17,0 | 16,8 |
| GB3-UGB2-UGB1 11 | GB3 | 0,01 x PBS, pH 4 | 19,8 | 9,5 | 16,8 |
| GB4-UGB2-UGB1 12 | GB4 | 0,01 x PBS, pH 4 | 13,9 | 9,5 | 16,8 |
| GB1-UGB2 13 | GB1 | 0,1 x PBS, pH 6 | 44,8 | 0,0 | 31,7 |
| GB5-UGB2-UGB1 14 | GB5 | 0,01 x PBS, pH 4 | 6,2 | 12,5 | 16,8 |
| UGB1-UGB2 15 | - | UGB1: 0,01 PBS, pH 4; UGB2: 0,01 PBS, pH 6,4 | 0,0 | 17,3 | 16,8 |
| GB1-UGB2 16 | GB1 | 0,1 x PBS, pH 6 | 44,8 | 0,0 | 31,7 |
| GB1-UGB2 17 | GB1 | 0,1 x PBS, pH 6 | 44,8 | 0,0 | 31,7 |
| GB1-UGB2 18 | GB1 | 0,1 x PBS, pH 6 | 44,8 | 0,0 | 31,7 |
| GB2-UGB2-UGB1 19 | GB2 | 0,01 x PBS, pH 4 | 30,8 | 0,0 | 14,9 |
| GB2-UGB2-UGB1 20 | GB2 | 0,01 x PBS, pH 4 | 0,6 | 14,9 | 15,2 |
| GB3-UGB2-UGB1 21 | GB3 | 0,01 x PBS, pH 4 | 29,9 | 0,0 | 22,8 |
| GB4-UGB2-UGB1 22 | GB4 | 0,01 x PBS, pH 4 | 28,9 | 0,0 | 19,6 |

**Tabelle 2:** Zusammensetzung und Synthese von Polymernetzwerken als Vorstufe eines Hydrogelmaterials

**Tabellen 3-1 und 3-2:** elektrische Eigenschaften der verschiedenen elektrisch leitfähigen Hydrogelmaterialien

Tabelle 3-1

| Hydrogelmaterialtyp | GB1-UGB2 01 | GB1-UGB2-UGB1 02 | GB1-UGB2-UGB1 03 | GB1-UGB2-UGB1 04 | GB2-UGB2 05 | GB2-UGB2-UGB1 06 | GB2-UGB2-UGB1 07 | GB2-UGB2-UGB108 |
|---|---|---|---|---|---|---|---|---|
| Relativer Quellungsgrad | 1,29 | 1,58 | 1,46 | 1,11 | 1,11 | 1,47 | 1,11 | 1,11 |
| P0 ($\mu$mol/ml) nach Quellung | 46 | 4 | 2 | 1 | 51 | 5 | 2 | 1 |
| P1 ($\mu$mol/ml) nach Quellung | 110 | 11 | 4 | 2 | 128 | 12 | 5 | 2 |
| P2 (mmol/(g/mol)) | 4,5 | 4,5 | 4,5 | 4,5 | 4,5 | 4,5 | 4,5 | 4,5 |
| P3 (1/A-2)*10^3 | -5,9 | -5,9 | -5,9 | -5,9 | 6,8 | 6,8 | 6,8 | 6,8 |
| geladener Polymerbaustei | GB 1 | GB 1 | GB 1 | GB 1 | GB 2 | GB 2 | GB 2 | GB 2 |
| Ladungsspeicherkapazität (mC/ml) | 3040 | 2350 | 1811 | 1505 | 2730 | 3040 | 2140 | 1894 |
| $\pm$ S.D. | 28,28 | 14,14 | 21,21 | 128,69 | 14,14 | 56,57 | 28,28 | 82,02 |
| Impedanz bei 0,01 Hz ($\Omega$) | 30 | 42 | 81 | 93 | 42 | 36 | 68 | 74 |
| $\pm$ S.D. | 1,52 | 2,12 | 4,06 | 4,65 | 2,08 | 1,82 | 3,39 | 3,70 |

Tabelle 3-2

| Hydrogelmaterialtyp | GB2-UGB2-UGB1 09 | GB2-UGB2-UGB1 10 | GB3-UGB2-UGB1 11 | GB4-UGB2-UGB1 12 | GB1-UGB2 13 | GB5-UGB2-UGB1 14 | UGB1-UGB2 15 | GB1-UGB2 16 | GB1-UGB2 17 | Cryogel GB1-UGB2 18 |
|---|---|---|---|---|---|---|---|---|---|---|
| Relativer Quellungsgrad | 1,50 | 1,30 | 1,43 | 1,39 | 1,25 | 1,25 | 1,25 | 1,25 | 1,60 | 1,60 |
| P0 ($\mu$mol/ml) nach Quellung | 24 | 1 | 25 | 26 | 34 | 6 | 0 | 34 | 26 | 26 |
| P1 ($\mu$mol/ml) nach Quellung | 60 | 2 | 43 | 9 | 81 | 9 | 0 | 81 | 81 | 81 |
| P2 (mmol/(g/mol)) | 4,5 | 4,5 | 3,2 | 0,9 | 4,5 | 1,8 | 0,0 | 4,5 | 4,5 | 4,5 |
| P3 (1/A-2)*10^3 | 6,8 | 6,8 | -2,5 | 0,7 | -5,9 | -6,8 | -2,8 | -5,9 | -5,9 | -5,9 |
| geladener Polymerbaustein | GB 2 | GB 2 | GB 3 | GB4 | GB 1 | GB5 | UGB1 | GB 1 | GB 1 | GB 1 |
| Ladungsspeicherkapazität (mC/ml) | 1819 | 1659 | 1222 | 910 | 2887 | 2453 | 921 | 37 | 2853 | 30 |
| ± S.D. | 176,65 | 37,43 | 16,97 | 59,40 | 122,20 | 70,24 | 22,48 | 2,14 | 94,52 | 0,90 |
| Impedanz bei 0,01 Hz ($\Omega$) | 92 | 103 | 93 | 150 | 53 | 62 | 167 | 8946 | 53 | 10636 |
| ± S.D. | 3,36 | 6,18 | 25,51 | 20,39 | 0,91 | 1,37 | 8,46 | 1143,08 | 1,26 | 1112,39 |

# Tabelle 4-1

## Sequestrierung in % (24 h)

| P0 (µmol/ml) nach Quellung | | | 40 | 24 | | |
|---|---|---|---|---|---|---|
| P1 (µmol/ml) nach Quellung | | | 84 | 60 | | |
| P2 (mmol/(g/mol)) | | | 4,5 | 4,5 | | |
| P3 (1/A-2)*10^3 | | | 6,8 | 6,8 | | |
| Hydrogelmaterialtyp | | | GB2-UGB2-UGB1 19 | GB2-UGB2-UGB1 09 | | |
| Protein | Klasse | Pp *10-6 [1/Å2] | kein PEDOT | 500 mV | 0 mV | -500 mV |
| SDF-1α | Positive geladen | 1139 | 98,20 | 25,29 | 72,31 | 47,21 |
| | | | | 54,72 | 13,17 | 4,58 |
| MCP-1 | | 1083 | 95,50 | 9,13 | 33,41 | 0,00 |
| | | | | 64,57 | 28,30 | 0,00 |
| FGF-2 | | 942 | 85,00 | 80,90 | 72,67 | 80,77 |
| | | | | 14,21 | 11,19 | 1,59 |
| NGF-β | | 350 | 100,00 | 70,01 | 94,06 | 74,97 |
| | | | | 21,01 | 2,69 | 2,94 |
| IL-4 | | 315 | 91,20 | 63,56 | 72,15 | 60,43 |
| | | | | 29,73 | 13,58 | 2,41 |
| IL-8 | | 247 | 96,70 | 48,09 | 70,78 | 35,35 |
| | | | | 19,02 | 13,17 | 14,20 |
| PDGF-BB | | 175 | 100,00 | | 86,59 | |
| | | | | | 12,13 | |
| IL-10 | Neutral | 27 | 88,40 | 86,46 | 51,59 | 52,69 |
| | | | | 10,97 | 21,16 | 15,85 |
| TNF-α | | -44 | 87,60 | 65,23 | 46,31 | 15,13 |
| | | | | 30,09 | 20,97 | 4,99 |
| IFN-γ | | -83 | 90,10 | | 66,36 | |
| | | | | | 19,43 | |
| VEGF | | -128 | 79,40 | 0,00 | 64,75 | 0,00 |
| | | | | 0,00 | 18,23 | 0,00 |
| GM-CSF | Negativ geladen | -394 | 43,30 | 59,24 | 49,93 | 17,88 |
| | | | | 31,29 | 19,10 | 4,67 |
| MIP-1β | | -427 | 88,30 | 45,46 | 62,78 | 0,00 |
| | | | | 37,34 | 16,07 | 0,00 |
| EGF | | -814 | 33,50 | 66,99 | 37,15 | 0,00 |
| | | | | 22,56 | 25,41 | 0,00 |

**Tabellen 4-1 bis 4-4:** Bindung bioaktiver Substanzen durch elektrisch leitfähige Hydrogelmaterialien

# Tabelle 4-2

## Sequestrierung in % (24 h)

| P0 (µmol/ml) nach Quellung | | | 1 | | 1 | |
|---|---|---|---|---|---|---|
| P1 (µmol/ml) nach Quellung | | | 2 | | 2 | |
| P2 (mmol/(g/mol)) | | | 4,5 | | 4,5 | |
| P3 (1/A-2)*10^3 | | | 6,8 | | 6,8 | |
| Hydrogelmaterialtyp | | | GB2-UGB2-UGB1 20 | GB2-UGB2-UGB1 10 | | |
| Protein | Klasse | Pp *10-6 [1/Å2] | kein PEDOT | 500 mV | 0 mV | -500 mV |
| SDF-1α | Positive geladen | 1139 | 60,50 | 31,95 | 76,88 | 19,68 |
| | | | | 18,08 | 6,64 | 24,71 |
| MCP-1 | | 1083 | 78,20 | 49,61 | 59,52 | 27,30 |
| | | | | 9,46 | 13,50 | 26,96 |
| FGF-2 | | 942 | 41,60 | 51,27 | 86,10 | 70,33 |
| | | | | 14,44 | 5,89 | 2,79 |
| NGF-β | | 350 | 79,00 | 7,93 | 60,89 | 25,77 |
| | | | | 20,01 | 17,74 | 30,45 |
| IL-4 | | 315 | 45,80 | 61,88 | 69,12 | 48,65 |
| | | | | 12,48 | 16,04 | 24,17 |
| IL-8 | | 247 | 67,80 | 17,06 | 39,94 | 3,69 |
| | | | | 10,10 | 19,34 | 25,96 |
| PDGF-BB | | 175 | 70,10 | 0,00 | 66,38 | 0,00 |
| | | | | 0,00 | 14,25 | 0,00 |
| IL-10 | Neutral | 27 | 46,10 | 0,00 | 61,17 | 0,00 |
| | | | | 0,00 | 11,02 | 0,00 |
| TNF-α | | -44 | 25,10 | 0,00 | 45,94 | 0,00 |
| | | | | 0,00 | 22,62 | 0,00 |
| IFN-γ | | -83 | 64,40 | | 48,30 | |
| | | | | | 18,56 | |
| VEGF | | -128 | 54,00 | 0,00 | 42,42 | 0,00 |
| | | | | 0,00 | 19,03 | 0,00 |
| GM-CSF | Negativ geladen | -394 | 0,00 | 78,40 | 77,79 | 44,91 |
| | | | | 5,47 | 6,72 | 25,37 |
| MIP-1β | | -427 | 63,90 | 26,49 | 78,08 | 28,26 |
| | | | | 16,78 | 5,85 | 25,05 |
| EGF | | -814 | 21,00 | 64,83 | 69,23 | 42,78 |
| | | | | 10,66 | 18,64 | 23,67 |

# Tabelle 4-3

## Sequestrierung in % (24 h)

| P0 (µmol/ml) nach Quellung | | | 45 | 25 | | |
|---|---|---|---|---|---|---|
| P1 (µmol/ml) nach Quellung | | | 81 | 43 | | |
| P2 (mmol/(g/mol)) | | | 3,2 | 3,2 | | |
| P3 (1/A-2)*10^3 | | | -2,5 | -2,5 | | |
| Hydrogelmaterialtyp | | | GB3-UGB2-UGB1 21 | GB3-UGB2-UGB1 11 | | |
| Protein | Klasse | Pp *10-6 [1/Å2] | kein PEDOT | 500 mV | 0 mV | -500 mV |
| SDF-1α | Positive geladen | 1139 | 90,20 | 36,82 | 70,85 | 43,66 |
| | | | | 18,93 | 11,96 | 1,29 |
| MCP-1 | | 1083 | 85,10 | 81,86 | 80,59 | 41,09 |
| | | | | 2,09 | 5,47 | 8,64 |
| FGF-2 | | 942 | 58,10 | 72,49 | 87,01 | 75,62 |
| | | | | 4,43 | 5,01 | 1,82 |
| NGF-β | | 350 | 84,80 | 31,85 | 84,57 | 36,58 |
| | | | | 5,65 | 1,55 | 9,16 |
| IL-4 | | 315 | 78,10 | 76,76 | 78,10 | 68,41 |
| | | | | 0,26 | 6,98 | 4,13 |
| IL-8 | | 247 | 87,20 | 64,68 | 83,85 | 37,80 |
| | | | | 8,60 | 5,78 | 3,25 |
| PDGF-BB | | 175 | 72,60 | 20,63 | 86,18 | 12,97 |
| | | | | 21,61 | 9,59 | 18,34 |
| IL-10 | Neutral | 27 | 33,00 | 58,02 | 72,43 | 4,11 |
| | | | | 7,43 | 7,49 | 16,39 |
| TNF-α | | -44 | 44,80 | 66,33 | 70,98 | 16,58 |
| | | | | 3,30 | 5,90 | 14,23 |
| IFN-γ | | -83 | 76,50 | | | |
| VEGF | | -128 | 41,10 | 0,00 | 67,46 | 0,00 |
| | | | | 0,00 | 33,81 | 0,00 |
| GM-CSF | Negativ geladen | -394 | 14,60 | 74,42 | 64,04 | 60,48 |
| | | | | 11,59 | 7,60 | 5,68 |
| MIP-1β | | -427 | 62,00 | 53,68 | 75,09 | 0,00 |
| | | | | 3,30 | 6,13 | 0,00 |
| EGF | | -814 | 6,00 | 72,68 | 40,45 | 0,00 |
| | | | | 1,82 | 26,51 | 0,00 |

# Tabelle 4-4

## Sequestrierung in % (24 h)

| P0 (µmol/ml) nach Quellung | | | 33 | | 26 | |
|---|---|---|---|---|---|---|
| P1 (µmol/ml) nach Quellung | | | 12 | | 9 | |
| P2 (mmol/(g/mol)) | | | 0,9 | | 0,9 | |
| P3 (1/A-2)*10^3 | | | 0,7 | | 0,7 | |
| Hydrogelmaterialtyp | | | GB4-UGB2-UGB1 22 | GB4-UGB2-UGB1 12 | | |
| Protein | Klasse | Pp *10-6 [1/Å2] | kein PEDOT | 500 mV | 0 mV | -500 mV |
| SDF-1α | Positive geladen | 1139 | 58,90 | 0,00 | 93,28 | 15,56 |
| | | | | 0,00 | 4,25 | 5,10 |
| MCP-1 | | 1083 | 78,70 | 75,65 | 76,42 | 67,74 |
| | | | | 13,21 | 6,75 | 11,66 |
| FGF-2 | | 942 | 41,00 | 67,27 | 91,78 | 68,40 |
| | | | | 13,83 | 2,52 | 3,48 |
| NGF-β | | 350 | 71,30 | 0,00 | 95,89 | 0,00 |
| | | | | 0,00 | 2,32 | 0,00 |
| IL-4 | | 315 | 48,30 | 55,55 | 78,40 | 73,33 |
| | | | | 5,15 | 9,47 | 5,73 |
| IL-8 | | 247 | 73,00 | 46,30 | 75,79 | 58,38 |
| | | | | 33,42 | 11,06 | 17,55 |
| PDGF-BB | | 175 | 59,00 | 0,00 | 96,86 | 20,54 |
| | | | | 0,00 | 2,18 | 4,45 |
| IL-10 | Neutral | 27 | 17,10 | 32,94 | 71,16 | 29,25 |
| | | | | 21,02 | 5,40 | 17,09 |
| TNF-α | | -44 | 46,50 | 49,24 | 73,96 | 38,62 |
| | | | | 12,25 | 5,88 | 16,43 |
| IFN-γ | | -83 | 70,00 | | | |
| | | | | | | |
| VEGF | | -128 | 56,90 | 0,00 | 76,52 | 0,00 |
| | | | | 0,00 | 13,13 | 0,00 |
| GM-CSF | Negativ geladen | -394 | 9,50 | 63,21 | 64,24 | 74,97 |
| | | | | 3,76 | 5,24 | 11,51 |
| MIP-1β | | -427 | 53,00 | 28,60 | 68,33 | 14,49 |
| | | | | 21,72 | 10,07 | 18,71 |
| EGF | | -814 | 8,50 | 55,89 | 17,59 | 0,00 |
| | | | | 22,89 | 11,65 | 0,00 |

# Tabelle 5-1

## Freisetzung in % (8h)

| P0 (µmol/ml) nach Quellung | | | 24 | | |
|---|---|---|---|---|---|
| P1 (µmol/ml) nach Quellung | | | 60 | | |
| P2 (mmol/(g/mol)) | | | 4,5 | | |
| P3 (1/A-2)*10^3 | | | 6,8 | | |
| Hydrogelmaterialtyp | | | GB2-UGB2-UGB1 09 | | |
| Protein | Klasse | Pp *10-6 [1/Å2] | 500 mV | 0 mV | -500 mV |
| SDF-1α | Positive geladen | 1139 | 0,05 | 0,00 | 0,00 |
| | | | 0,07 | 0,00 | 0,00 |
| MCP-1 | | 1083 | 0,18 | 0,76 | 2,70 |
| | | | 0,09 | 0,24 | 0,05 |
| FGF-2 | | 942 | 0,00 | 0,00 | 0,00 |
| | | | 0,00 | 0,00 | 0,00 |
| NGF-β | | 350 | 0,00 | 0,00 | 0,00 |
| | | | 0,00 | 0,00 | 0,00 |
| IL-4 | | 315 | 0,22 | 0,33 | 0,36 |
| | | | 0,06 | 0,23 | 0,06 |
| IL-8 | | 247 | 0,21 | 0,48 | 3,25 |
| | | | 0,08 | 0,30 | 0,15 |
| PDGF-BB | | 175 | 0,00 | 0,00 | 0,00 |
| | | | 0,00 | 0,00 | 0,00 |
| IL-10 | Neutral | 27 | 0,00 | 0,09 | 0,00 |
| | | | 0,00 | 0,05 | 0,00 |
| TNF-α | | -44 | 0,00 | 1,39 | 5,26 |
| | | | 0,00 | 1,35 | 3,01 |
| IFN-γ | | -83 | 0,00 | 0,00 | 0,77 |
| | | | 0,00 | 0,00 | 1,09 |
| VEGF | | -128 | 0,08 | 0,14 | 1,56 |
| | | | 0,02 | 0,07 | 1,08 |
| GM-CSF | Negativ geladen | -394 | 0,22 | 2,33 | 10,41 |
| | | | 0,03 | 2,41 | 0,47 |
| MIP-1β | | -427 | 0,17 | 0,87 | 8,77 |
| | | | 0,24 | 0,57 | 1,13 |
| EGF | | -814 | 0,86 | 9,12 | 67,18 |
| | | | 0,47 | 3,94 | 49,77 |

**Tabellen 5-1 bis 5-4:** Freisetzung bioaktiver Substanzen durch elektrisch leitfähige Hydrogelmaterialeien bei verschiedenen elektrischen Potentialen

# Tabelle 5-2

## Freisetzung in % (8h)

| PO (μmol/ml) nach Quellung | | | 1 | | |
|---|---|---|---|---|---|
| P1 (μmol/ml) nach Quellung | | | 2 | | |
| P2 (mmol/(g/mol)) | | | 4,5 | | |
| P3 (1/A-2)*10^3 | | | 6,8 | | |
| Hydrogelmaterialtyp | | | GB2-UGB2-UGB1 10 | | |
| Protein | Klasse | Pp *10-6 [1/Å2] | 500 mV | 0 mV | -500 mV |
| SDF-1α | Positive geladen | 1139 | 0,23 | 0,75 | 0,00 |
|  |  |  | 0,33 | 0,73 | 0,00 |
| MCP-1 |  | 1083 | 0,32 | 4,35 | 4,58 |
|  |  |  | 0,29 | 2,29 | 1,26 |
| FGF-2 |  | 942 | 0,69 | 0,31 | 2,55 |
|  |  |  | 0,67 | 0,20 | 0,47 |
| NGF-β |  | 350 | 0,55 | 0,54 | 5,26 |
|  |  |  | 0,78 | 0,15 | 3,20 |
| IL-4 |  | 315 | 0,17 | 0,60 | 1,71 |
|  |  |  | 0,14 | 0,24 | 0,16 |
| IL-8 |  | 247 | 1,18 | 11,69 | 26,33 |
|  |  |  | 0,87 | 5,97 | 12,08 |
| PDGF-BB |  | 175 | 1,55 | 0,98 | 0,00 |
|  |  |  | 2,19 | 1,69 | 0,00 |
| IL-10 | Neutral | 27 | 0,02 | 0,03 | 0,14 |
|  |  |  | 0,02 | 0,00 | 0,12 |
| TNF-α |  | -44 | 0,88 | 1,70 | 53,20 |
|  |  |  | 0,85 | 1,05 | 19,68 |
| IFN-γ |  | -83 | 10,50 | 17,50 | 57,33 |
|  |  |  | 5,29 | 7,86 | 19,10 |
| VEGF |  | -128 | 2,05 | 1,29 | 8,56 |
|  |  |  | 2,64 | 0,80 | 2,68 |
| GM-CSF | Negativ geladen | -394 | 0,11 | 0,38 | 5,90 |
|  |  |  | 0,04 | 0,08 | 0,02 |
| MIP-1β |  | -427 | 0,56 | 0,64 | 2,96 |
|  |  |  | 0,17 | 0,33 | 0,58 |
| EGF |  | -814 | 0,08 | 1,96 | 72,62 |
|  |  |  | 0,05 | 0,52 | 49,49 |

# Tabelle 5-3

## Freisetzung in % (8h)

| PO (µmol/ml) nach Quellung | | | 25 | | |
|---|---|---|---|---|---|
| P1 (µmol/ml) nach Quellung | | | 43 | | |
| P2 (mmol/(g/mol)) | | | 3,2 | | |
| P3 (1/A-2)*10^3 | | | -2,5 | | |
| Hydrogelmaterialtyp | | | GB3-UGB2-UGB1 11 | | |
| Protein | Klasse | Pp *10-6 [1/Å2] | 500 mV | 0 mV | -500 mV |
| SDF-1α | | 1139 | 0,81 | 1,63 | 2,42 |
| | | | 0,16 | 1,52 | 1,46 |
| MCP-1 | | 1083 | 0,22 | 1,33 | 2,10 |
| | | | 0,01 | 0,25 | 1,33 |
| FGF-2 | Positive geladen | 942 | 0,00 | 0,01 | 0,08 |
| | | | 0,00 | 0,03 | 0,11 |
| NGF-β | | 350 | 0,00 | 0,21 | 0,00 |
| | | | 0,00 | 0,36 | 0,00 |
| IL-4 | | 315 | 1,34 | 2,23 | 1,66 |
| | | | 0,58 | 1,41 | 0,59 |
| IL-8 | | 247 | 0,30 | 1,43 | 1,88 |
| | | | 0,02 | 0,28 | 0,84 |
| PDGF-BB | | 175 | 0,00 | 0,00 | 0,00 |
| | | | 0,00 | 0,00 | 0,00 |
| IL-10 | | 27 | 0,08 | 0,30 | 0,43 |
| | | | 0,02 | 0,03 | 0,27 |
| TNF-α | Neutral | -44 | 0,07 | 0,83 | 2,85 |
| | | | 0,07 | 0,16 | 2,82 |
| IFN-γ | | -83 | | | |
| | | | | | |
| VEGF | | -128 | 1,70 | 2,96 | 13,74 |
| | | | 0,77 | 2,29 | 11,01 |
| GM-CSF | | -394 | 0,50 | 4,35 | 12,29 |
| | | | 0,20 | 0,24 | 9,19 |
| MIP-1β | Negativ geladen | -427 | 0,93 | 2,92 | 3,47 |
| | | | 0,25 | 0,18 | 1,39 |
| EGF | | -814 | 1,19 | 94,10 | 100,00 |
| | | | 0,40 | 139,88 | 128,28 |

# Tabelle 5-4

## Freisetzung in % (8h)

| P0 (µmol/ml) nach Quellung | | | 26 | | |
|---|---|---|---|---|---|
| P1 (µmol/ml) nach Quellung | | | 9 | | |
| P2 (mmol/(g/mol)) | | | 0,9 | | |
| P3 (1/A-2)*10^3 | | | 0,7 | | |
| Hydrogelmaterialtyp | | | GB4-UGB2-UGB1 12 | | |
| Protein | Klasse | Pp *10-6 [1/Å2] | 500 mV | 0 mV | -500 mV |
| SDF-1α | | 1139 | 0,06 | 0,23 | 0,21 |
| | | | 0,08 | 0,17 | 0,01 |
| MCP-1 | | 1083 | 0,16 | 1,35 | 2,08 |
| | | | 0,01 | 0,40 | 1,02 |
| FGF-2 | Positive geladen | 942 | 0,00 | 0,02 | 0,13 |
| | | | 0,00 | 0,02 | 0,07 |
| NGF-β | | 350 | 0,00 | 0,09 | 0,02 |
| | | | 0,00 | 0,08 | 0,03 |
| IL-4 | | 315 | 0,29 | 0,83 | 1,05 |
| | | | 0,05 | 0,20 | 0,54 |
| IL-8 | | 247 | 0,16 | 1,18 | 3,68 |
| | | | 0,09 | 0,70 | 1,68 |
| PDGF-BB | | 175 | 0,09 | 0,08 | 0,05 |
| | | | 0,12 | 0,07 | 0,07 |
| IL-10 | | 27 | 0,04 | 0,26 | 0,37 |
| | | | 0,01 | 0,05 | 0,16 |
| TNF-α | Neutral | -44 | 0,20 | 0,75 | 1,38 |
| | | | 0,12 | 0,34 | 0,65 |
| IFN-γ | | -83 | | | |
| | | | | | |
| VEGF | | -128 | 0,08 | 0,60 | 0,94 |
| | | | 0,03 | 0,42 | 0,63 |
| GM-CSF | | -394 | 0,22 | 3,65 | 5,76 |
| | | | 0,17 | 0,28 | 2,93 |
| MIP-1β | Negativ geladen | -427 | 0,10 | 2,17 | 6,34 |
| | | | 0,14 | 0,67 | 4,18 |
| EGF | | -814 | 3,44 | 73,54 | 100,00 |
| | | | 3,45 | 22,11 | |

# Tabelle 6

## PC12 Kultur unter Verwendung von Cryogelen GB1-UGB2 17

| Zellumfang (µm/Zelle) | | | | |
|---|---|---|---|---|
| t (d) | 5 | | 7 | |
| | Mittelwert | S.D. | Mittelwert | S.D. |
| NGF-β ohne | 7,92 | 1,32 | 4,32 | 0,46 |
| NGF-β im Medium | 18,49 | 1,53 | 19,58 | 4,34 |
| NGF-β im Gel | 10,25 | 0,51 | 9,55 | 1,67 |
| NGF-β im Gel, reduziertes Medium | 13,1 | 2,14 | 17,77 | 4,45 |
| Zellzahl (Zellen/cm²) | | | | |
| t (d) | 5 | | 7 | |
| | Mittelwert | S.D. | Mittelwert | S.D. |
| NGF-β ohne | 919,17 | 304,79 | 4553,24 | 727,33 |
| NGF-β im Medium | 1590,24 | 336,16 | 1934,93 | 686,48 |
| NGF-β im Gel | 2138,08 | 359,01 | 2852,43 | 1222,45 |
| NGF-β im Gel, reduziertes Medium | 1501,98 | 365,23 | 1523,63 | 265,07 |

**Tabelle** 6: Biokompatibilität von elektrisch leitfähigen Hydrogelmaterialien gegenüber PC12 Zellen und daraus differenzierter Neuron-artigen Zellen

# Elektrische Eigenschaften

| | elektr. Charakt. | elektr. Charakt. | elektr. Charakt. | elektr. Charakt. | elektr. Charakt. | elektr. Charakt. | elektr. Charakt. | elektr. Charakt. | Multiplex | Multiplex | Multiplex | Multiplex |
|---|---|---|---|---|---|---|---|---|---|---|---|---|
| Netzwerk Typ | 1 | 2 | 3 | 4 | 5 | 6 | 7 | 8 | 9 | 10 | 11 | 12 |
| P0 (µmol/ml) after swelling | 46,00 | 4,00 | 2,00 | 1,00 | 51,00 | 5,00 | 2,00 | 1,00 | 24,00 | 1,00 | 25,00 | 26,00 |
| P1 (µmol/ml) before swelling | 142,00 | 17,00 | 5,70 | 1,70 | 143 | 18 | 6,10 | 1,80 | 91 | 3,00 | 61 | 12 |
| P1 (µmol/ml) after swelling | 110,00 | 11,00 | 4,00 | 2,00 | 128,00 | 12,00 | 5,00 | 2,00 | 60,00 | 2,00 | 43,00 | 9,00 |
| P2 (mmol/(g/mol)) | 4,5 | 4,5 | 4,5 | 4,5 | 4,5 | 4,5 | 4,5 | 4,5 | 4,5 | 4,5 | 3,2 | 0,9 |
| P3 *10^-3 (1/A^2) | -5,9 | -5,9 | -5,9 | -5,9 | 6,8 | 6,8 | 6,8 | 6,8 | 6,8 | 6,8 | -2,5 | 0,7 |
| Fractional charge | 0,32 | 0,32 | 0,32 | 0,32 | 0,26 | 0,26 | 0,26 | 0,26 | 0,26 | 0,26 | 0,30 | 0,30 |
| SC% | 5,00% | 3.3% (3.41%) | 3.3% (3.34%) | 3.3% (3.31%) | 2,60% | 3.3% (3.5%) | 3.3% (3.37%) | 3.3% (3.32%) | 3.3% (4.3%) | 3.3% (3.33%) | 3.3% (4.41%) | 3.3% (3.87%) |
| % Polymer Mal | 100,0% | 15,0% | 5,0% | 1,5% | 100% | 12% | 4% | 1,2% | 60% | 2% | 45% | 45% |
| Swelling degree | 1,29 | 1,58 | 1,46 | 1,11 | 1,11 | 1,47 | 1,11 | 1,11 | 1,50 | 1,30 | 1,43 | 1,39 |
| geladener Polymerbausteine | HEP | HEP | HEP | HEP | PSSA 3:1 | PSSA 3:1 | PSSA 3:1 | PSSA 3:1 | PSSA 3:1 | PSSA 3:1 | AEHS50% | AEHS10% |
| geladener Polymerbausteine | GB 1 | GB 1 | GB 1 | GB 1 | GB 2 | GB 2 | GB 2 | GB 2 | GB 2 | GB 2 | GB 3 | GB 4 |
| CSC (mC/ml) | 3040 | 2350 | 1811 | 1505 | 2730 | 3040 | 2140 | 1894 | 1819 | 1659 | 1222 | 910 |
| ± S.D. | 28,28 | 14,14 | 21,21 | 128,69 | 14,14 | 56,57 | 28,28 | 82,02 | 176,65 | 37,43 | 16,97 | 59,40 |
| Impedanz bei 0,01 Hz (Ω) | 30 | 42 | 81 | 93 | 84 | 63 | 80 | 94 | 92 | 103 | 93 | 150 |
| ± S.D. | 1,52 | 2,12 | 4,06 | 4,65 | 2,95 | 2,41 | 5,52 | 4,55 | 3,36 | 6,18 | 25,51 | 20,39 |

| NGF Aufnahme und Freisetzung | NGF Aufnahme und Freisetzung | Kontrolle elektr. Charakt. | | | Cryogele | |
|---|---|---|---|---|---|---|
| 13 | 14 | 15 | 16 | | 17 | 18 |
| 33,60 | 5,58 | 0,00 | 33,60 | | | |
| 101 | 17 | 0 | 101 | | 101 | 101 |
| 81,00 | 9,00 | 0,00 | 81,00 | | | |
| 4,51 | 1,78 | 0 | 4,51 | | 4,51 | 4,51 |
| -5,9 | -6,75 | -2,82 | -5,9 | | -5,9 | -5,9 |
| 0,32 | 0,32 | 0,00 | 0,32 | | 0,32 | 0,32 |
| 3,30% | 3.3% (3.41%) | 3,30% | 3,30% | | 3,30% | 3,30% |
| | 15% | 0% | | | | |
| 1,25 | 1,58 | 1,25 | 1,25 | | | |
| HEP | 6-O,N DHEP | PEG-PEG | HEP | | HEP | HEP |
| GB 1 | GB5 | UGB1 | GB 1 | | GB 1 | GB 1 |
| 2887 | 2453 | 921 | 37 | | 2853 | 30 |
| 122,20 | 70,24 | 22,48 | 2,14 | | 94,52 | 0,90 |
| 53 | 62 | 167 | 8946 | | 53 | 10636 |
| 0,91 | 1,37 | 8,46 | 1143,08 | | 1,26 | 1112,39 |

# Elektrische Eigenschaften 1

| | | | | | | | | | | | | | |
|---|---|---|---|---|---|---|---|---|---|---|---|---|---|
| Swelling degree | | 1,293333 | 1,578629 | 1,455293 | 1,11 | 1,113333 | 1,472055 | 1,111092 | 1,111092 | 1,5049835 | 1,3 | 1,43 | 1,39 | 1,25 |
| P0 (µmol/ml) after swelling | | 45,695888 | 4,370881 | 1,622354 | 0,664865 | 51,46708 | 4,89112 | 2,178937 | 0,653411 | 24,12784 | 0,93 | 25,393007 | 26,123741 | 0 |
| P1 (µmol/ml) after swelling | | 109,79 | 10,77 | 3,92 | 1,53 | 128,44 | 12,23 | 5,49 | 1,62 | 60,47 | 2,31 | 42,66 | 8,63 | 0 |
| P2 (mmol/(g/mol)) | | 4,5 | 4,5 | 4,5 | 4,5 | 4,5 | 4,5 | 4,5 | 4,5 | 4,5 | 4,5 | 3,23 | 0,94 | 0 |
| P3 *10^-3 (1/A^2) | | -5,9 | -5,9 | -5,9 | -5,9 | 6,8 | 6,8 | 6,8 | 6,8 | 6,8 | 6,8 | -2,5 | 0,7 | -2,82 |
| Charged polymer | HEP | HEP | HEP | HEP | PSSA 3:1 | PSSA 3:1 | PSSA 3:1 | PSSA 3:1 | PSSA 3:1 | PSSA 3:1 | AEHS50% | AEHS10% | PEG-PEG |
| CSC (mC/ml) | | 3040 | 2350 | 1811 | 1505 | 2730 | 3040 | 2140 | 1894 | 1818,6667 | 1659,3333 | 1222 | 910 | 920,6666667 |
| ± S.D. | | 28,28427 | 14,14214 | 21,2132 | 128,6934 | 14,14214 | 56,56854 | 28,28427 | 82,02439 | 176,65031 | 37,434387 | 16,970563 | 59,39697 | 22,4796204 |
| Impedanz bei 0,01 Hz (Ω) | | 30,322 | 42,341 | 81,192 | 93,012 | 41,508 | 36,341 | 67,777 | 74,03 | 91,916667 | 102,871 | 93,293 | 149,529 | 166,8166667 |
| ± S.D. | | 1,5161 | 2,11705 | 4,0596 | 4,6506 | 2,0754 | 1,81705 | 3,38885 | 3,7015 | 3,3589459 | 6,1818815 | 25,510998 | 20,391545 | 8,461349203 |

EP 4 531 059 A2

# Elektrische Eigenschaften 1

EP 4 531 059 A2

| | Bulk | Cryo | undoped control | Bulk | Cryo | P1 low | |
|---|---|---|---|---|---|---|---|
| Swelling degree | 1,25 | 1,6 | 1,25 | 1,25 | 1,6 | 1,578629221 | 1,25 |
| P1 (µmol/ml) after swelling | 81 | 81 | 0 | 81 | 81 | 10,77 | 9 |
| P2 (mmol/(g/mol)) | 4,5 | 4,5 | 0 | 4,5 | 4,5 | 4,5 | 1,78 |
| P3 *10^-3 (1/A^2) | -5,9 | -5,9 | -2,82 | -5,9 | -5,9 | -5,9 | -6,75 |
| Charged polymer | HEP | HEP | PEG-PEG | HEP | HEP | HEP | 6-O,N DHEP |
| CSC (mC/ml) | 36,66666667 | 30,13333333 | 920,6666667 | 2886,666667 | 2853,333333 | 2350 | 2453,333333 |
| ± S.D. | 2,138535324 | 0,901849951 | 22,4796204 | 122,2020185 | 94,51631253 | 14,14214 | 70,23769169 |
| Impedanz at 0,01 Hz (Ω) | 8946 | 10635,66667 | 166,8166667 | 53,381 | 52,62433333 | 42,341 | 61,76 |
| ± S.D. | 1143,083986 | 1112,387672 | 8,461349203 | 0,912167748 | 1,263355189 | 2,11705 | 1,372269653 |
| | w/o PEDOT | w/o PEDOT | | | | | |

# Sequestrierung in % (24

| | | | | 19 | 9 | | | 20 | 10 | | | 21 | 11 | | | 22 | 12 | | |
|---|---|---|---|---|---|---|---|---|---|---|---|---|---|---|---|---|---|---|---|
| | Swelling degree | | | | 1,50 | | | | 1,30 | | | | 1,43 | | | | 1,39 | | |
| | P0 (µmol/ml) after swelling | | | | 24 | | | | 1 | | | | 25 | | | | 26 | | |
| | P1 (µmol/ml) after swelling | | | 70 | 60 | | | 2 | 2 | | | 70 | 43 | | | 15 | 9 | | |
| | P2 (mmol/(g/mol)) | | | 4,5 | 4,5 | | | 4,5 | 4,5 | | | 3,2 | 3,2 | | | 0,9 | 0,9 | | |
| | P3 (1/A-2)*10^3 | | | 6,8 | 6,8 | | | 6,8 | 6,8 | | | -2,5 | -2,5 | | | 0,7 | 0,7 | | |
| | beladener Polymerbaustein | | | PSSA3:1 | PSSA3:1 | | | PSSA3:1 | PSSA3:1 | | | AEHS50 | AEHS50% | | | AEHS10 | AEHS10% | | |
| IEP | Protein | Klasse | Charge | no PEDOT | 500 mV | 0 mV | -500 mV | no PEDOT | 500 mV | 0 mV | -500 mV | no PEDOT | 500 mV | 0 mV | -500 mV | no PEDOT | 500 mV | 0 mV | -500 mV |
| 10,1 | SDF-1α | | 1139 | 98,2 | 25,3 | 72,3 | 47,2 | 60,5 | 32,0 | 76,9 | 19,7 | 90,2 | 36,8 | 70,9 | 43,7 | 58,9 | 0,0 | 93,3 | 15,6 |
| 9,4 | MCP-1 | | 1083 | 95,5 | 9,1 | 33,4 | 0,0 | 78,2 | 49,6 | 59,5 | 27,3 | 85,1 | 81,9 | 80,6 | 41,1 | 78,7 | 75,7 | 76,4 | 67,7 |
| 9,58 | FGF-2 | | 942 | 85,0 | 80,9 | 72,7 | 80,8 | 41,6 | 51,3 | 86,1 | 70,3 | 58,1 | 72,5 | 87,0 | 75,6 | 41,0 | 67,3 | 91,8 | 68,4 |
| 9 | NGF-β | | 350 | 100,0 | 70,0 | 94,1 | 75,0 | 79,0 | 7,9 | 60,9 | 25,8 | 84,8 | 31,9 | 84,6 | 36,6 | 71,3 | 0,0 | 95,9 | 0,0 |
| 9,3 | IL-4 | | 315 | 91,2 | 63,6 | 72,2 | 60,4 | 45,8 | 61,9 | 69,1 | 48,6 | 78,1 | 76,8 | 78,1 | 68,4 | 48,3 | 55,5 | 78,4 | 73,3 |
| 9,2 | IL-8 | | 247 | 96,7 | 48,1 | 70,8 | 35,4 | 67,8 | 17,1 | 39,9 | 3,7 | 87,2 | 64,7 | 83,8 | 37,8 | 73,0 | 46,3 | 75,8 | 58,4 |
| 9,38 | PDGF-BB | Positive gel | 175 | 100,0 | | 86,6 | | 70,1 | 0,0 | 66,4 | 0,0 | 72,6 | 20,6 | 86,2 | 13,0 | 59,0 | 0,0 | 96,9 | 20,5 |
| 7,6 | IL-10 | | 27 | 88,4 | 86,5 | 51,6 | 52,7 | 46,1 | 0,0 | 61,2 | 0,0 | 33,0 | 58,0 | 72,4 | 4,1 | 17,1 | 32,9 | 71,2 | 29,3 |
| 7 | TNF-α | | -44 | 87,6 | 65,2 | 46,3 | 15,1 | 25,1 | 0,0 | 45,9 | 0,0 | 44,8 | 66,3 | 71,0 | 16,6 | 46,5 | 49,2 | 74,0 | 38,6 |
| 9,5 | IFN-γ | | -83 | 90,1 | | 66,4 | | 64,4 | | 48,3 | | 76,5 | | | | 70,0 | | | |
| 7,6 | VEGF | Neutral | -128 | 79,4 | 0,0 | 64,7 | 0,0 | 54,0 | 0,0 | 42,4 | 0,0 | 41,1 | 0,0 | 67,5 | 0,0 | 56,9 | 0,0 | 76,5 | 0,0 |
| 5,2 | GM-CSF | | -394 | 43,3 | 59,2 | 49,9 | 17,9 | 0,0 | 78,4 | 77,8 | 44,9 | 14,6 | 74,4 | 64,0 | 60,5 | 9,5 | 63,2 | 64,2 | 75,0 |
| 4,8 | MIP-1β | | -427 | 88,3 | 45,5 | 62,8 | 0,0 | 63,9 | 26,5 | 78,1 | 28,3 | 62,0 | 53,7 | 75,1 | 0,0 | 53,0 | 28,6 | 68,3 | 14,5 |
| 4,8 | EGF | Negativ gel | -814 | 33,5 | 67,0 | 37,1 | 0,0 | 21,0 | 64,8 | 69,2 | 42,8 | 6,0 | 72,7 | 40,4 | 0,0 | 8,5 | 55,9 | 17,6 | 0,0 |

# Sequestrierung in % ±S.D. (24 h)

| | ID | Analyt | | | | | | | | | | | | | | | | | |
|---|---|---|---|---|---|---|---|---|---|---|---|---|---|---|---|---|---|---|---|
| Positive geladen | 10,1 | SDF-1α | 1139 | 98,20 | 25,29 | 72,31 | 47,21 | 60,50 | 31,95 | 76,88 | 19,68 | 90,20 | 36,82 | 70,85 | 43,66 | 58,90 | 0,00 | 93,28 | 15,56 |
| | | | | | 54,72 | 13,17 | 4,58 | | 18,08 | 6,64 | 24,71 | | 18,93 | 11,96 | 1,29 | | 0,00 | 4,25 | 5,10 |
| | 9,4 | MCP-1 | 1083 | 95,50 | 9,13 | 33,41 | 0,00 | 78,20 | 49,61 | 59,52 | 27,30 | 85,10 | 81,86 | 80,59 | 41,09 | 78,70 | 75,65 | 76,42 | 67,74 |
| | | | | | 64,57 | 28,30 | 0,00 | | 9,46 | 13,50 | 26,96 | | 2,09 | 5,47 | 8,64 | | 13,21 | 6,75 | 11,66 |
| | 9,58 | FGF-2 | 942 | 85,00 | 80,90 | 72,67 | 80,77 | 41,60 | 51,27 | 86,10 | 70,33 | 58,10 | 72,49 | 87,01 | 75,62 | 41,00 | 67,27 | 91,78 | 68,40 |
| | | | | | 14,21 | 11,19 | 1,59 | | 14,44 | 5,89 | 2,79 | | 4,43 | 5,01 | 1,82 | | 13,83 | 2,52 | 3,48 |
| | 9 | NGF-β | 350 | 100,00 | 70,01 | 94,06 | 74,97 | 79,00 | 7,93 | 60,89 | 25,77 | 84,80 | 31,85 | 84,57 | 36,58 | 71,30 | 0,00 | 95,89 | 0,00 |
| | | | | | 21,01 | 2,69 | 2,94 | | 20,01 | 17,74 | 30,45 | | 5,65 | 1,55 | 9,16 | | 0,00 | 2,32 | 0,00 |
| | 9,3 | IL-4 | 315 | 91,20 | 63,56 | 72,15 | 60,43 | 45,80 | 61,88 | 69,12 | 48,65 | 78,10 | 76,76 | 78,10 | 68,41 | 48,30 | 55,55 | 78,40 | 73,33 |
| | | | | | 29,73 | 13,58 | 2,41 | | 12,48 | 16,04 | 24,17 | | 0,26 | 6,98 | 4,13 | | 5,15 | 9,47 | 5,73 |
| | 9,2 | IL-8 | 247 | 96,70 | 48,09 | 70,78 | 35,35 | 67,80 | 17,06 | 39,94 | 3,69 | 87,20 | 64,68 | 83,85 | 37,80 | 73,00 | 46,30 | 75,79 | 58,38 |
| | | | | | 19,02 | 13,17 | 14,20 | | 10,10 | 19,34 | 25,96 | | 8,60 | 5,78 | 3,25 | | 33,42 | 11,06 | 17,55 |
| | 9,38 | PDGF-BB | 175 | 100,00 | | 86,59 | | 70,10 | 0,00 | 66,38 | 0,00 | 72,60 | 20,63 | 86,18 | 12,97 | 59,00 | 0,00 | 96,86 | 20,54 |
| | | | | | | 12,13 | | | 0,00 | 14,25 | 0,00 | | 21,61 | 9,59 | 18,34 | | 0,00 | 2,18 | 4,45 |
| Neutral | 7,6 | IL-10 | 27 | 88,40 | 86,46 | 51,59 | 52,69 | 46,10 | 0,00 | 61,17 | 0,00 | 33,00 | 58,02 | 72,43 | 4,11 | 17,10 | 32,94 | 71,16 | 29,25 |
| | | | | | 10,97 | 21,16 | 15,85 | | 0,00 | 11,02 | 0,00 | | 7,43 | 7,49 | 16,39 | | 21,02 | 5,40 | 17,09 |
| | 7 | TNF-α | -44 | 87,60 | 65,23 | 46,31 | 15,13 | 25,10 | 0,00 | 45,94 | 0,00 | 44,80 | 66,33 | 70,98 | 16,58 | 46,50 | 49,24 | 73,96 | 38,62 |
| | | | | | 30,09 | 20,97 | 4,99 | | 0,00 | 22,62 | 0,00 | | 3,30 | 5,90 | 14,23 | | 12,25 | 5,88 | 16,43 |
| | 9,5 | IFN-γ | -83 | 90,10 | | 66,36 | | 64,40 | | 48,30 | | 76,50 | | | | 70,00 | | | |
| | | | | | | 19,43 | | | | 18,56 | | | | | | | | | |
| | 7,6 | VEGF | -128 | 79,40 | 0,00 | 64,75 | 0,00 | 54,00 | 0,00 | 42,42 | 0,00 | 41,10 | 0,00 | 67,46 | 0,00 | 56,90 | 0,00 | 76,52 | 0,00 |
| | | | | | 0,00 | 18,23 | 0,00 | | 0,00 | 19,03 | 0,00 | | 0,00 | 33,81 | 0,00 | | 0,00 | 13,13 | 0,00 |
| Negativ geladen | 5,2 | GM-CSF | -394 | 43,30 | 59,24 | 49,93 | 17,88 | 0,00 | 78,40 | 77,79 | 44,91 | 14,60 | 74,42 | 64,04 | 60,48 | 9,50 | 63,21 | 64,24 | 74,97 |
| | | | | | 31,29 | 19,10 | 4,67 | | 5,47 | 6,72 | 25,37 | | 11,59 | 7,60 | 5,68 | | 3,76 | 5,24 | 11,51 |
| | 4,8 | MIP-1β | -427 | 88,30 | 45,46 | 62,78 | 0,00 | 63,90 | 26,49 | 78,08 | 28,26 | 62,00 | 53,68 | 75,09 | 0,00 | 53,00 | 28,60 | 68,33 | 14,49 |
| | | | | | 37,34 | 16,07 | 0,00 | | 15,78 | 5,85 | 25,05 | | 3,30 | 6,13 | 0,00 | | 21,72 | 10,07 | 18,71 |
| | 4,8 | EGF | -814 | 33,50 | 66,99 | 37,15 | 0,00 | 21,00 | 64,83 | 69,23 | 42,78 | 6,00 | 72,68 | 40,45 | 0,00 | 8,50 | 55,89 | 17,59 | 0,00 |
| | | | | | 22,56 | 25,41 | 0,00 | | 10,66 | 18,64 | 23,67 | | 1,82 | 26,51 | 0,00 | | 22,89 | 11,65 | 0,00 |

# Freisetzung 1

| Freisetzung in % (8h) | | | | 9 | | | 10 | | | 11 | | | 12 | | |
|---|---|---|---|---|---|---|---|---|---|---|---|---|---|---|---|
| Swelling degree | | | | 1,50 | | | 1,30 | | | 1,43 | | | 1,39 | | |
| P0 (µmol/ml) after swelling | | | | 24 | | | 1 | | | 25 | | | 26 | | |
| P1 (µmol/ml) after swelling | | | | 60 | | | 2 | | | 43 | | | 9 | | |
| P2 (mmol/(g/mol)) | | | | 4,50 | | | 4,50 | | | 3,23 | | | 0,94 | | |
| P3 (1/A-2)*10^3 | | | | 6,8 | | | 6,8 | | | -2,5 | | | 0,7 | | |
| geladener Polymerbaustein | | | | PSSA3:1 | | | PSSA3:1 | | | AEHS50% | | | AEHS10% | | |
| IEP | Protein | Klasse | Charge/SA (x10^6) (1/A^2) | 500 mV | 0 mV | -500 mV | 500 mV | 0 mV | -500 mV | 500 mV | 0 mV | -500 mV | 500 mV | 0 mV | -500 mV |
| 10,1 | SDF-1α | Positiv geladen | 1139 | 0,0 | 0,0 | 0,0 | 0,2 | 0,8 | 0,0 | 0,8 | 1,6 | 2,4 | 0,1 | 0,2 | 0,2 |
| 9,4 | MCP-1 | | 1083 | 0,2 | 0,8 | 2,7 | 0,3 | 4,4 | 4,6 | 0,2 | 1,3 | 2,1 | 0,2 | 1,4 | 2,1 |
| 9,58 | FGF-2 | | 942 | 0,0 | 0,0 | 0,0 | 0,7 | 0,3 | 2,6 | 0,0 | 0,0 | 0,1 | 0,0 | 0,0 | 0,1 |
| 9 | NGF-β | | 350 | 0,0 | 0,0 | 0,0 | 0,6 | 0,5 | 5,3 | 0,0 | 0,2 | 0,0 | 0,0 | 0,1 | 0,0 |
| 9,3 | IL-4 | | 315 | 0,2 | 0,3 | 0,4 | 0,2 | 0,6 | 1,7 | 1,3 | 2,2 | 1,7 | 0,3 | 0,8 | 1,1 |
| 9,2 | IL-8 | | 247 | 0,2 | 0,5 | 3,2 | 1,2 | 11,7 | 26,3 | 0,3 | 1,4 | 1,9 | 0,2 | 1,2 | 3,7 |
| 9,38 | PDGF-BB | | 175 | 0,0 | 0,0 | 0,0 | 1,6 | 1,0 | 0,0 | 0,0 | 0,0 | 0,0 | 0,1 | 0,1 | 0,1 |
| 7,6 | IL-10 | Neutral | 27 | 0,0 | 0,1 | 0,0 | 0,0 | 0,0 | 0,1 | 0,1 | 0,3 | 0,4 | 0,0 | 0,3 | 0,4 |
| 7 | TNF-α | | -44 | 0,0 | 1,4 | 5,3 | 0,9 | 1,7 | 53,2 | 0,1 | 0,8 | 2,9 | 0,2 | 0,8 | 1,4 |
| 9,5 | IFN-γ | | -83 | 0,0 | 0,0 | 0,8 | 10,5 | 17,5 | 57,3 | | | | | | |
| 7,6 | VEGF | | -128 | 0,1 | 0,1 | 1,6 | 2,1 | 1,3 | 8,6 | 1,7 | 3,0 | 13,7 | 0,1 | 0,6 | 0,9 |
| 5,2 | GM-CSF | Negativ geladen | -394 | 0,2 | 2,3 | 10,4 | 0,1 | 0,4 | 5,9 | 0,5 | 4,3 | 12,3 | 0,2 | 3,7 | 5,8 |
| 4,8 | MIP-1β | | -427 | 0,2 | 0,9 | 8,8 | 0,6 | 0,6 | 3,0 | 0,9 | 2,9 | 3,5 | 0,1 | 2,2 | 6,3 |
| 4,8 | EGF | | -814 | 0,9 | 9,1 | 67,2 | 0,1 | 2,0 | 72,6 | 1,2 | 94,1 | 100,0 | 3,4 | 73,5 | 100,0 |

# Freisetzung 1

| Freisetzung in % ±S.D. (8h) | | | | | | | | | | | | | | |
|---|---|---|---|---|---|---|---|---|---|---|---|---|---|---|
| 10,1 | SDF-1α | | 1139 | 0,05 | 0,00 | 0,00 | 0,23 | 0,75 | 0,00 | 0,81 | 1,63 | 2,42 | 0,06 | 0,23 | 0,21 |
| | | | | 0,07 | 0,00 | 0,00 | 0,33 | 0,73 | 0,00 | 0,16 | 1,52 | 1,46 | 0,08 | 0,17 | 0,01 |
| 9,4 | MCP-1 | | 1083 | 0,18 | 0,76 | 2,70 | 0,32 | 4,35 | 4,58 | 0,22 | 1,33 | 2,10 | 0,16 | 1,35 | 2,08 |
| | | | | 0,09 | 0,24 | 0,05 | 0,29 | 2,29 | 1,26 | 0,01 | 0,25 | 1,33 | 0,01 | 0,40 | 1,02 |
| 9,58 | FGF-2 | Positive geladen | 942 | 0,00 | 0,00 | 0,00 | 0,69 | 0,31 | 2,55 | 0,00 | 0,01 | 0,08 | 0,00 | 0,02 | 0,13 |
| | | | | 0,00 | 0,00 | 0,00 | 0,67 | 0,20 | 0,47 | 0,00 | 0,03 | 0,11 | 0,00 | 0,02 | 0,07 |
| 9 | NGF-β | | 350 | 0,00 | 0,00 | 0,00 | 0,55 | 0,54 | 5,26 | 0,00 | 0,21 | 0,00 | 0,00 | 0,09 | 0,02 |
| | | | | 0,00 | 0,00 | 0,00 | 0,78 | 0,15 | 3,20 | 0,00 | 0,36 | 0,00 | 0,00 | 0,08 | 0,03 |
| 9,3 | IL-4 | | 315 | 0,22 | 0,33 | 0,36 | 0,17 | 0,60 | 1,71 | 1,34 | 2,23 | 1,66 | 0,29 | 0,83 | 1,05 |
| | | | | 0,06 | 0,23 | 0,06 | 0,14 | 0,24 | 0,16 | 0,58 | 1,41 | 0,59 | 0,05 | 0,20 | 0,54 |
| 9,2 | IL-8 | | 247 | 0,21 | 0,48 | 3,25 | 1,18 | 11,69 | 26,33 | 0,30 | 1,43 | 1,88 | 0,16 | 1,18 | 3,68 |
| | | | | 0,08 | 0,30 | 0,15 | 0,87 | 5,97 | 12,08 | 0,02 | 0,28 | 0,84 | 0,09 | 0,70 | 1,68 |
| 9,38 | PDGF-BB | | 175 | 0,00 | 0,00 | 0,00 | 1,55 | 0,98 | 0,00 | 0,00 | 0,00 | 0,00 | 0,09 | 0,08 | 0,05 |
| | | | | 0,00 | 0,00 | 0,00 | 2,19 | 1,69 | 0,00 | 0,00 | 0,00 | 0,00 | 0,12 | 0,07 | 0,07 |
| 7,6 | IL-10 | Neutral | 27 | 0,00 | 0,09 | 0,00 | 0,02 | 0,03 | 0,14 | 0,08 | 0,30 | 0,43 | 0,04 | 0,26 | 0,37 |
| | | | | 0,00 | 0,05 | 0,00 | 0,02 | 0,00 | 0,12 | 0,02 | 0,03 | 0,27 | 0,01 | 0,05 | 0,16 |
| 7 | TNF-α | | -44 | 0,00 | 1,39 | 5,26 | 0,88 | 1,70 | 53,20 | 0,07 | 0,83 | 2,85 | 0,20 | 0,75 | 1,38 |
| | | | | 0,00 | 1,35 | 3,01 | 0,85 | 1,05 | 19,68 | 0,07 | 0,16 | 2,82 | 0,12 | 0,34 | 0,65 |
| 9,5 | IFN-γ | | -83 | 0,00 | 0,00 | 0,77 | 10,50 | 17,50 | 57,33 | | | | | | |
| | | | | 0,00 | 0,00 | 1,09 | 5,29 | 7,86 | 19,10 | | | | | | |
| 7,6 | VEGF | | -128 | 0,08 | 0,14 | 1,56 | 2,05 | 1,29 | 8,56 | 1,70 | 2,96 | 13,74 | 0,08 | 0,60 | 0,94 |
| | | | | 0,02 | 0,07 | 1,08 | 2,64 | 0,80 | 2,68 | 0,77 | 2,29 | 11,01 | 0,03 | 0,42 | 0,63 |
| 5,2 | GM-CSF | Negativ geladen | -394 | 0,22 | 2,33 | 10,41 | 0,11 | 0,38 | 5,90 | 0,50 | 4,35 | 12,29 | 0,22 | 3,65 | 5,76 |
| | | | | 0,03 | 2,41 | 0,47 | 0,04 | 0,08 | 0,02 | 0,20 | 0,24 | 9,19 | 0,17 | 0,28 | 2,93 |
| 4,8 | MIP-1β | | -427 | 0,17 | 0,87 | 8,77 | 0,56 | 0,64 | 2,96 | 0,93 | 2,92 | 3,47 | 0,10 | 2,17 | 6,34 |
| | | | | 0,24 | 0,57 | 1,13 | 0,17 | 0,33 | 0,58 | 0,25 | 0,18 | 1,39 | 0,14 | 0,67 | 4,18 |
| 4,8 | EGF | | -814 | 0,86 | 9,12 | 67,18 | 0,08 | 1,96 | 72,62 | 1,19 | 94,10 | 100,00 | 3,44 | 73,54 | 100,00 |
| | | | | 0,47 | 3,94 | 49,77 | 0,05 | 0,52 | 49,49 | 0,40 | 139,88 | 128,28 | 3,45 | 22,11 | out of dete |

EP 4 531 059 A2

38

**Patentansprüche**

1. Elektrisch leitfähiges Hydrogelmaterial (1), aufweisend ein aus anionisch geladenen Bausteinen (3) und ungeladenen Bausteinen ausgebildetes Polymernetzwerk (2), welches in seiner Zusammensetzung anhand von mindestens drei die anionisch geladenen Bausteine (3) definierenden Parametern, ausgewählt aus einer Gruppe von Parametern P0, P1, P2, P3, konfigurierbar ist, wobei der Parameter P0 einem Wert aus der Anzahl der ionisierten, anionischen Gruppen, unter Annahme einer 30%igen Ionisierung aller anionischen Gruppen, je Volumeneinheit des unter physiologischen Bedingungen gequollenen Hydrogelmaterials (1) entspricht, der Parameter P1 einem Wert aus der Anzahl der stark anionischen Gruppen, mit einem intrinsischen pKs-Wert kleiner als 2,5, je Volumeneinheit des unter physiologischen Bedingungen gequollenen Hydrogelmaterials (1) entspricht, der Parameter P2 einem Wert aus der Anzahl der stark anionischen Gruppen, mit einem intrinsischen pKs-Wert kleiner als 2,5, je Wiederholeinheit geteilt durch die Molmasse der Wiederholeinheit entspricht und der Parameter P3 einem Wert zur Beschreibung der Amphiphilie der anionisch geladenen Bausteine (3) entspricht, und eine elektrisch leitfähige Komponente (5), welche in dem Polymernetzwerk (2) inkorporiert ist, wobei eine elektrische Leitfähigkeit, ein elektrischer Widerstand und/oder eine elektrische Ladungsspeicherkapazität des Hydrogelmaterials (1) durch Parameterwerte einer Parameterkonfiguration des Hydrogelmaterials (1) vorgebbar ist **dadurch gekennzeichnet, dass** die anionisch geladenen Bausteine (3) ausgewählt sind aus einer Gruppe, enthaltend Poly(Acrylsäure-co-4-acrylamidomethylbenzensulfonsäure), Poly(Acrylsäure-co-Acrylamidoethansulfonsäure), Poly(Acrylsäure-co-Acrylamidoethanhydrogensulfat), Poly(4-Styrensulfonsäure-co-Maleinsäure), sulfatierte Glykosaminoglykane, insbesondere Heparin, selektiv desulfatierte Heparinderivate, Heparansulfat, Chondroitinsulfat, Keratansulfat und Dermatansulfat, und dass die ungeladenen Bausteine Aminogruppen oder Thiolgruppen enthaltende Polymere oder Vernetzermoleküle (4) mit mindestens zwei Aminogruppen oder Thiolgruppen sind, wobei die geladenen und ungeladenen Bausteine vernetzt sind zu dem Polymernetzwerk (2), erhältlich durch eine Aktivierung von Carboxylgruppen der Poly(Acrylsäure-co-4-acrylamidomethylbenzensulfonsäure) und/oder der Poly(Acrylsäure-co-Acrylamidoethansulfonsäure) und/oder der Poly(Acrylsäure-co-Acrylamidoethanhydrogensulfat) und/oder Poly(4-Styrensulfonsäure-co-Maleinsäure), und/oder sulfatierten Glykosaminoglykanen, insbesondere Heparin, und/oder selektiv desulfatierten Heparinderivaten und/oder Heparansulfat, und/oder Chondroitinsulfat und/oder Keratansulfat und/oder Dermatansulfat mit EDC/Sulfo-NHS und entweder eine direkte Vernetzung mit den Aminogruppen enthaltenden Polymeren oder den Vernetzermolekülen (4) mit den mindestens zwei Aminogruppen jeweils unter Amidbildung oder einer Funktionalisierung der aktivierten Carboxylgruppen mittels bifunktionellen Vernetzermolekülen (4), welche jeweils eine Aminogruppe und eine zu einer Michael-Typ-Addition fähige Gruppe enthalten, und die anschließende Vernetzung mit den Thiolgruppen enthaltenden Polymeren oder den Vernetzermolekülen (4) mit den mindestens zwei Thiolgruppen jeweils über eine Michael-Typ-Addition.

2. Elektrisch leitfähiges Hydrogelmaterial (1), nach Anspruch 1, **dadurch gekennzeichnet, dass** die elektrische Impedanz des Hydrogelmaterials (1), gemessen bei einer Frequenz von 0,01 Hz, im Bereich von 150 $\Omega$ bis 10 $\Omega$ variierbar ist, wobei die Parameterwerte einer Parameterkonfiguration vorzugsweise so gewählt sind, dass eine elektrische Impedanz von 30 $\Omega$ erreicht ist.

3. Elektrisch leitfähiges Hydrogelmaterial (1), nach einem der Ansprüche 1 oder 2, **dadurch gekennzeichnet, dass** die elektrische Ladungsspeicherkapazität des Hydrogelmaterials (1) im Bereich von 900 mC/ml bis 4000 mC/ml variierbar ist, wobei die Parameterwerte einer Parameterkonfiguration vorzugsweise so gewählt sind, dass eine Ladungsspeicherkapazität von 2480 mC/ml erreicht ist.

4. Elektrisch leitfähiges Hydrogelmaterial (1) nach einem der Ansprüche 1 bis 3, **dadurch gekennzeichnet, dass** die elektrisch leitfähige Komponente (5) ein $\Pi$-konjugiertes, elektrisch leitfähiges Polymer oder eine Polymerzusammensetzung aus Polypyrrol, Polyanilin, Polythiophen und/oder Poly(3,4-ethylendoxythiophen) (PEDOT) ist.

5. Elektrisch leitfähiges Hydrogelmaterial (1) nach Anspruch 1, **dadurch gekennzeichnet, dass** die Aminogruppen und Thiolgruppen enthaltenden Polymere als ungeladene Bausteine ausgewählt sind aus der Klasse der Polyethylenglykole (PEG), Poly(2-oxazoline) (POX), Polyvinylpyrrolidone (PVP), Polyvinylalkohole (PVA) und/oder Polyarylamide (PAM) und dass die Aminogruppen oder Thiolgruppen enthaltenden Vernetzermoleküle (4) nichtpolymere, bifunktionelle Vernetzermoleküle (4) sind.

6. Elektrisch leitfähiges Hydrogelmaterial (1) nach einem der Ansprüche 1 bis 5, **dadurch gekennzeichnet, dass** als ungeladene Bausteine Polymere mit konjugierten enzymatisch spaltbaren Peptiden, die entweder Lysin oder Cystein als reaktive Aminosäure in der Peptidsequenz aufweisen, für die Polymernetzwerkbildung genutzt werden.

7. Elektrisch leitfähiges Hydrogelmaterial (1) nach Anspruch 1 bis 6, **dadurch gekennzeichnet, dass** die enzymatisch spaltbaren Peptide durch humane oder bakterielle Proteasen, insbesondere Matrixmetalloproteasen (MMPs), Cathepsine, Elastasen, Aureolysin und/oder Blutgerinnungsenzyme, spaltbar sind.

8. Elektrisch leitfähiges Hydrogelmaterial (1) nach Anspruch 1 bis 7, **dadurch gekennzeichnet, dass** bioaktive und/oder antiadhäsive Moleküle mit einer Aminogruppe oder Carboxylgruppe und/oder zellinstruktive Peptide über Lysin oder Cystein in einer Sequenz an den geladenen Bausteinen Poly(Acrylsäure-co-4-acrylamidomethylbenzensulfonsäure) und/oder Poly(Acrylsäure-co-Acrylamidoethansulfonsäure) und/oder Poly(Acrylsäure-co-Acrylamidoethanhydrogensulfat) und/oder Poly(4-Styrensulfonsäure-co-Maleinsäure), und/oder sulfatierten Glykosaminoglykanen wie Heparin und/oder selektiv desulfatierten Heparinderivaten und/oder Heparansulfat und/oder Chondroitinsulfat und/oder Keratansulfat und/oder Dermatansulfat oder an deren Derivaten mit zur Michael-Typ-Addition fähigen Gruppen unter Ausbildung einer kovalenten Bindung an das Hydrogelnetzwerk angebunden sind.

9. Elektrisch leitfähiges Hydrogelmaterial (1) nach Anspruch 8, **dadurch gekennzeichnet, dass** die bioaktiven Moleküle antimikrobielle Substanzen, zum Beispiel Antibiotika oder Antiseptika, oder pharmazeutische Wirkstoffe sind.

10. Elektrisch leitfähiges Hydrogelmaterial (1) nach einem der Ansprüche 1 bis 9, **dadurch gekennzeichnet, dass** für den Parameter P0 ein Wert in einem Bereich von 0 bis 80 $\mu$mol/ml, für den Parameter P1 ein Wert in einem Bereich von 0 bis 150 $\mu$mol/ml, für den Parameter P2 ein Wert in einem Bereich von 0 bis 10 mmol/(g/mol) und für den Parameter P3 ein Wert in einem Bereich von $-7 \times 10^{-3}$ bis $7 \times 10^{-3}$ A$^{-2}$ vorgebbar ist.

11. Elektrisch leitfähiges Hydrogelmaterial (1) nach einem der Ansprüche 1 bis 10, **dadurch gekennzeichnet, dass** das Hydrogelmaterial (1) ein Speichermodul von 0,2 kPa bis 22 kPa aufweist.

12. Verwendung eines elektrisch leitfähigen Hydrogelmaterials (1) nach einem der Ansprüche 1 bis 11 zum *in vivo* Faktormanagement zur Kontrolle der Angiogenese, bei Immunerkrankungen, Krebserkrankungen, Diabetis, neurodegenerativen Erkrankungen, Morbus Crohn, Colitis Ulcerosa, Multiple Sklerose, Asthma, Rheumatoide Arthritis oder der kutanen Wundheilung und der Knochenregeneration.

13. Verwendung eines elektrisch leitfähigen Hydrogelmaterials (1) nach einem der Ansprüche 1 bis 11 zur elektrischen Stimulation von Zellen oder Geweben.

14. Verwendung eines elektrisch leitfähigen Hydrogelmaterials (1) nach einem der Ansprüche 1 bis 9 zur gezielten Aufreinigung von Proteinen aus Zelllysaten mikrobieller oder eukaryotischer Herkunft.

15. Verwendung eines elektrisch leitfähigen Hydrogelmaterials (1) nach einem der Ansprüche 1 bis 11, zur *in vitro* Zellkultur und Organkultur von induziert pluripotenten Stammzellen (iPS-) sowie weiteren nicht iPS zuzuordnenden Stammzellen und Vorläuferzellen, primären, von Patienten gewonnen Zellen, immortalisierten Zelllinien sowie Herzgewebe, Muskelgewebe, Nierengewebe, Lebergewebe und Nervengewebe.

Fig. 1

Fig. 2

## Fig. 3

Nicht-poröses Hydrogelmaterial

Poröses Hydrogelmaterial

**EP 4 531 059 A2**

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- WO 2018162009 A2
- US 20190390068 A1
- US 9299476 B2